# EUROPEAN PATENT SPECIFICATION

(11) **EP 2 828 378 B1**
(45) Date of publication and mention of the grant of the patent: **17.02.2021**
(21) Application number: 13764726.9
(22) Date of filing: 14.03.2013
(51) Int. Cl.: A61L 27/38, A61L 27/60, C12N 5/02, C12N 5/071, A61K 35/36

(54) **CELL SUSPENSION AND USE THEREOF**
ZELLSUSPENSION UND VERWENDUNG DAVON
SUSPENSION CELLULAIRE ET SON UTILISATION

(30) Priority: 22.03.2012 US 201261614115 P; 22.03.2012 US 201261614112 P
(43) Date of publication of application: 28.01.2015
(73) Proprietor: Avita Medical Ltd, Valencia, CA 91355 (US)
(72) Inventor: QUICK, Andrew, Valencia, CA 91355 (US); DOLPHIN, William, Topanga, CA 90290 (US)
(74) Representative: Cooley (UK) LLP
(86) International application number: PCT/US2013/031316
(87) International publication number: WO 2013/142254

(56) References cited:
- WO-A1-98/56897
- WO-A1-02/062358
- US-A1- 2002 048 563
- US-A1- 2005 272 147
- US-A1- 2007 184 033
- US-A1- 2007 286 880
- US-A1- 2010 035 815
- US-A1- 2010 255 052
- US-A1- 2011 070 646

## Description

### FIELD OF THE INVENTION

This invention relates to a simple, rapid and cost effective method for preparing a cell suspension, particularly a suspension comprising viable epithelial cells such as undifferentiated/progenitor cells and/or differentiated cells. Such suspension is useful for treating a patient in need of an epithelium-related procedure. Device for such preparation and use of the suspension thereof are also provided.

### BACKGROUND

Tissue regeneration in humans is extremely limited and constitutes a major challenge to the repair of damaged organ function. Wound treatment is a typical area where tissue regeneration is required. Wounds (lacerations or openings) in mammalian tissue can result in tissue disruption and coagulation of the microvasculature at the wound face. Repair of such tissue represents an orderly, controlled cellular response to injury. All soft tissue wounds, regardless of size, heal in a similar manner. The mechanisms of tissue growth and repair are biologic systems wherein cellular proliferation and angiogenesis occur in the presence of an oxygen gradient. The sequential morphological and structural changes, which occur during tissue repair have been characterized in great detail and have, in some instances, been quantified. See Hunt, T. K., et al., "Coagulation and macrophage stimulation of angiogenesis and wound healing," The surgical wound, pp. 1-18, ed. F. Dineen & G. Hildrick-Smith (Lea & Febiger, Philadelphia: 1981).

Tissue regeneration in various organs, such as the skin or the heart depends on connective tissue restoring blood supply and enabling residual organ-specific cells such as keratinocytes or muscle cells to reestablish organ integrity. Thus, a relevant function of the mesenchymal cells, e.g., the fibroblasts or, in addition, the endothelial cells of vasculature, is secretion of factors enhancing the healing process, e.g., factors promoting formation of new blood vessels (angiogenesis) or factors promoting re-epithelialization by proliferating and migrating keratinocytes.

The cellular morphology of a wound consists of three distinct zones. The central avascular wound space is oxygen deficient, acidotic and hypercarbic, and has high lactate levels. Adjacent to the wound spate is a gradient zone of local ischemia, which is populated by dividing fibroblasts. Behind the leading zone is an area of active collagen synthesis characterized by mature fibroblasts and numerous newly formed capillaries (i.e., neovascularization). While new blood vessel growth (angiogenesis) is necessary for the healing of wound tissue, angiogenic agents generally are unable to fulfill the long-felt need of providing the additional biosynthetic effects of tissue repair. In addition to acute wound (e.g., burn or laceration caused by trauma), artificially created wound (e.g., in a graft donor site, aesthetic indication, plastic procedure or dermal treatment), chronic wound (e.g., venous or diabetic ulcers) and other indications such scar remodeling, glabrous skin loss injuries, pigmentation issues, vitiligo, leucoderma and cosmetic rejuvenation procedures also require rapid and efficient therapeutics. Despite the need for more rapid healing of wounds (e.g., severe burns, surgical incisions, lacerations and other trauma), to date there has been only limited success in accelerating wound healing with pharmacological agents.

The primary goal in the conventional treatment of wounds is to achieve wound closure. Open cutaneous wounds represent one major category of wounds. This category includes acute surgical and traumatic, e.g., chronic ulcers, burn wounds, as well as chronic wounds such as neuropathic ulcers, pressure sores, arterial and venous (stasis) or mixed arterio-venous ulcers, and diabetic ulcers. Open cutaneous wounds routinely heal by a process comprising six major components: i) inflammation, ii) fibroblast proliferation, blood vessel proliferation, iv) connective tissue synthesis, v) epithelialization, and vi) wound contraction. Wound healing is impaired when these components, either individually or as a whole, do not function properly. Numerous factors can affect wound healing, including malnutrition, infection, pharmacological agents (e.g., cytotoxic drugs and corticosteroids), diabetes, and advanced age. See Hunt et al., in Current Surgical Diagnosis & Treatment (Way; Appleton & Lange), pp. 86-98 (1988).

Skin wounds that do not readily heal can cause the subject considerable physical, emotional, and social distress as well as great financial expense. See, e.g., Richey et al., Annals of Plastic Surgery 23(2): 159-65 (1989). Indeed, wounds that fail to heal properly finally may require aggressive surgical treatments such as autologous skin grafting (where sheets of skin are grafted) or cultured dermis grafting. For example, cultured epithelial autograft (CEA) procedures take skin cells from the patient to grow new skin cells in sheets in a laboratory. The new sheets are used as grafts. However, the take rate of these grafts is not satisfactory. See, e.g., Sood et al., Journal of Burn Care Research 31(4):559-68 (2010). Newer grafting procedures combine CEA with a matrix for more support. For example, currently available as cultured or engineered dermis are products having different matrices into which fibroblasts are incorporated, such as TransCyte® and Dermagraft®. However, these products are not efficient in inducing epithelialization in large wounds. Cultured/engineered skin incorporating epidermal cells and fibroblasts are available as Apligraf® (NOVARTIS Pharma) and VivoDerm® (Bristol-Myers Squibb). However, there are problems regarding the affinity between cultured epidermal layer and dermal layer, and insufficiency in clinical effect obtainable.
WO 02/062358 A1 (2002-08-15) reports a method and/or device for producing a transplantable cellular suspension of living tissue for grafting to a patient. In applying the method and/or in using the device donor tissue is harvested, subjected to a cell dissociation treatment, cells for grafting back to a patient are collected and dispersed in a solution that is for immediate dispersion over the recipient graft site.

A need for improved wound healing, and more broadly, tissue regeneration technique exists. The present invention provides an autologous cellular suspension suitable for application on various recipient sites, which can be used without regard to the type or tissue of the wound or the nature of the patient population. Methods for preparing and/or using said suspension, as well as devices for preparation are also provided.

### SUMMARY OF THE INVENTION

The subject invention relates to a unique cell suspension composition for use in a method for treating a patient in need of an epithelium-related procedure, and a method for its preparation that is rapid, efficient and simple to prepare and apply. Exemplary methods are disclosed for treating a patient in need of an epithelium-related procedure using the unique cell suspension composition, which not only promote would healing/closure, but also tissue regeneration. The cell suspension compositions of the invention can be applied to any epithelium-related procedure to promote tissue regeneration, including epithelial tissues such as skin epithelium (e.g., glabrous epithelium), respiratory epithelium, vascular epithelium, glandular epithelium, corneal epithelium. An apparatus suitable for use in the method of preparation and/or treatment is also provided for reference. Use of the described device is helpful for practicing the method of the present disclosure, and has been found to significantly reduce the time used in alternative procedures and the complexity associated with the use of conventional grafting technologies. Furthermore, the cell suspension composition of the present invention provides live, functioning cells that can be presented across a large area in or on a patient's body, or into a matrix or scaffold or substrate in vitro or in situ on a recipient site. Clinicians would otherwise have limited or no access to such autologous epithelial cells.

For reference, there is provided a cell suspension for use in an epithelium-related procedure. The cell suspension includes a population of cells including viable cells a population of cells including viable cells derived from an epithelial tissue sample. The viable cells can be at various stages of differentiation and be functioning (e.g., performing their pre-programmed functions such as signaling, producing certain proteins, secreting certain factors, migration, or proliferation). The cells can include mature/differentiated cells as well as cells capable of dividing or differentiating. The viable cells can also be in a migratory and proliferative state. The population of cells, prior to being used in an epithelium-related procedure, are exposed to a condition selected from the group consisting of stress and an exogenous agent. The population of cells can exhibit a stress-induced characteristic. Such stress-induced characteristic can cause the cells to express healing process mediating protein(s). Exogenous agent(s) or component(s) such as heat shock protein(s), hyaluronic acid, platelet-enriched plasma, growth factor(s), cytokine(s), and/or adipose stem cells can be supplied to the population of cells. The population of cells, with or without the exogenously supplied agent(s), when used in said epithelium-related procedure and applied to a patient's recipient site in vivo, can promote treatment, healing, reconstructing, resurfacing, repigmentation and/or regeneration of epithelial tissues. The population of cells can also be seeded to a matrix or scaffold or substrate (in vitro or in situ), where the cells can, for example, grow to form an artificial tissue or organ.

In an aspect, the present invention provides a composition for use in an epithelium-related procedure, as defined in claim 1. The composition includes a population of cells including viable and functioning cells derived from an epithelial tissue sample. The viable cells are functioning (e.g., performing their pre-programmed functions such as signaling, producing certain proteins, secreting certain factors, migration, or proliferation). The viable cells can be at various stages of differentiation and include mature/differentiated cells that are functioning, as well as cells capable of dividing or differentiating. The cells can also be in a migratory and proliferative state. In a comparative example, the population of cells, prior to being used in an epithelium-related procedure, are exposed to a condition selected from the group consisting of stress and an exogenous agent. In a comparative example, the composition can include, for example, a heat shock protein expressed by the cells under stress, or an isolated or recombinant heat shock protein or a fragment thereof. The heat shock protein can be in an effective amount to promote skin healing in a patient in need thereof. In the embodiments of the invention, the composition includes an exogenous agent comprising hyaluronic acid. The composition may further include an exogenous or externally supplied agent or component such as platelet-enriched plasma, growth factor(s), cytokine(s), and/or adipose stem cells. The composition, when used in an epithelium-related procedure and applied to a patient's recipient site in vivo, can promote treatment, healing, reconstructing, resurfacing, repigmentation and/or regeneration of epithelial tissues. The composition can also be applied to a matrix or scaffold or substrate in vitro or in situ, where the cells can grow (e.g., be cultured in vitro or multiply in situ) to form, e.g., an artificial tissue or organ.

In another aspect, the present invention provides a method for preparing cells for use in an epithelium-related procedure, as defined in claim 6. The method includes: providing a population of cells including viable and functioning cells derived from an epithelial tissue sample; and exposing the population of cells to an exogenous agent comprising hyaluronic acid. In a comparative example, the method involves exposing the population of cells to a condition selected from the group consisting of stress and an exogenous agent. The viable cells can be at various stages of differentiation and include mature/differentiated cells that are functioning, as well as cells capable of dividing or differentiating. The cells can also be in a migratory and proliferative state. In comparative examples, the method includes inducing expression of a heat shock protein by the population of cells, thereby obtaining cells for use in an epithelium-related procedure that have an elevated level of said heat shock protein compared to a control level of said heat shock protein without said stress condition. In comparative examples, the exogenous agent can be one or more of hyaluronic acid, platelet-enriched plasma, growth factor(s), cytokine(s), and/or adipose stem cells.

The present disclosure provides, for reference, a method for preparing and using cells in an epithelium-related procedure. The method includes: applying, to a recipient site on a patient in need thereof, a population of cells including viable cells derived from an epithelial tissue sample; and providing to the population of cells a condition selected from the group consisting of stress and an exogenous agent to promote epithelial treatment, healing, reconstructing, resurfacing, repigmentation and/or regeneration in said patient. In some examples, the condition is stress which can induce the cells to express a heat shock protein, or an isolated or recombinant heat shock protein or a fragment thereof provided exogenously, in an effective amount to promote epithelial treatment, healing, reconstructing, resurfacing, repigmentation and/or regeneration in said patient. In some examples, the exogenous heat shock protein can be provided separately or in combination with the population of cells to the recipient site on the patient. In certain examples, the exogenous agent can be one or more of hyaluronic acid, platelet-enriched plasma, growth factor(s), cytokine(s), and/or adipose stem cells.

In certain embodiments, when the epithelial tissue sample is skin tissue sample, the viable cells can be derived from a dermal layer, epidermal layer, or both a dermal and an epidermal layer of the skin tissue sample, and the population of cells can comprise keratinocytes, melanocytes, fibroblasts, Langerhans cells, or any combination of the foregoing. In some embodiments, the population of cells can include skin stem cells and/or other skin cells.

The stress-induced characteristic, referred to herein, may be an elevated level of expression of a protein selected from the group consisting of Hsp90 and Hsp90α. The stress-induced characteristic can be associated with hypoxia, heat, mechanical trauma, nutrient deprivation, enzyme exposure, or other stress-inducing manipulation.

In certain embodiments, when the epithelial tissue sample is skin tissue sample, the epithelium-related procedure comprises skin wound treatment, skin tissue graft, skin aesthetic procedure, or dermal treatment. The skin wound can be acute wound, artificially created wound, or chronic wound. The epithelial tissue sample can also be obtained from respiratory epithelium, vascular epithelium, corneal epithelium, or glandular epithelium, and the cells derived therefrom can be used to treat corresponding epithelial conditions.

Other aspects and advantages of the invention will become apparent to those skilled in the art from the ensuing description, which proceeds with reference to the following illustrative drawings.

### BRIEF DESCRIPTION OF THE DRAWINGS

FIG. 1 illustrates a typical structure and composition of a skin tissue.
FIG. 2A illustrates a perspective view of an exemplary apparatus with lid open and a second member in place.
FIG. 2B illustrates a perspective view of the exemplary apparatus with lid open and the second member removed and inverted.
FIG. 3A illustrates a perspective view of a first member of the exemplary apparatus.
FIG. 3B illustrates a perspective rear view of the first member of the exemplary apparatus.
FIG. 4 illustrates a perspective view of the base of the exemplary apparatus.
FIG. 5 shows results of Cell Viability Assay of keratinocytes using different hyaluronic acid hydrogels.
FIG. 6 shows results of Trans-well Migration Assay of keratinocytes using different hyaluronic acid hydrogels.
FIG. 7 shows results of *In vitro* Scratch Assay of keratinocytes using different hyaluronic acid hydrogels.

### DETAILED DESCRIPTION OF THE INVENTION

Those skilled in the art will appreciate that the compositions and methods described herein are adaptable to variations and modifications other than those specifically described. The disclosure also includes all of the steps, features, compositions and compounds referred to or indicated in the specification, individually or collectively and any and all combinations or any two or more of the steps or features.

Throughout this specification and the claims that follow, unless the context requires otherwise, the word "comprise", or variations such as "comprises" or "comprising", will be understood to imply the inclusion of a stated integer or group of integers but not the exclusion of any other integer or group of integers.

Having regard to the above, this disclosure provides a unique method and device suitable for producing a cellular suspension of living tissue suitable for application to a patient in an epithelium-related procedure. In applying the method and/or in using the device, a donor tissue (e.g., skin epithelium such as glabrous epithelium, respiratory epithelium, vascular epithelium, corneal epithelium, and glandular epithelium) is harvested from a patient and subjected to a tissue dissociating means, and cells suitable for applying back to a recipient site of the same patient are collected. In some comparative examples, the cells so collected can be cultured and expanded in vitro before applying to a recipient site. The cells can also be seeded to a scaffold or matrix or substrate where the cells can grow and/or proliferate into a regenerated tissue or organ.

In the embodiments, the cells include stem cells (e.g., cells capable dividing or differentiating) and non-stem cells (e.g., cells at various differentiating stages including differentiated and functioning cells). A number of organs rely on undifferentiated stem and progenitor cells for tissue regeneration. However, it is unclear if resident stem cells are capable of regenerating the full mass of tissue required for a given injury. Furthermore, stem cells have not yet been identified for a number of tissues, and in those tissues in which stem cells have been identified, the factors required to induce their propagation and differentiation to acquire the fates of cells in these tissues are not fully understood. Thus, there is a need for methods of inducing well defined differentiated cells of known identity to contribute to cell replacement and tissue regeneration in vivo. The present invention, by including both stem cells and non-stem cells in a cell suspension, provides a means for improved tissue regeneration.

The cells may be presented in the form of a cell suspension (used interchangeably with "cellular suspension" herein) in a solution that is suitable for immediate dispersion (e.g., immediately after harvesting and/or filtering without in vitro culturing of the cells) over the recipient site. The cell suspension can be dispersed (immediately after harvesting or after in vitro culturing) to the recipient site alone or in combination with additional factor(s) such as heat shock protein(s), hyaluronic acid, platelet-enriched plasma, growth factor(s), and/or adipose stem cells, to facilitate, for example, wound healing, skin re-surfacing, or other epithelial treatments. The cell suspension can be dispersed via spraying, dripping, or any other application process. The cell suspension can also be injected directly into a tissue.

The subject matter disclosed herein has many advantages over the previously known tissue grafting techniques, some of which are illustrated as follows:
1. The subject invention provides a time efficient method for preparing a cellular suspension which can be readily supplied to a recipient site in a clinical setting. For example, cells can be quickly harvested and processed when needed at the time of surgery. This is achieved by the very short preparation time of the cells, allowing epithelial treatment to be performed peri-operatively or in the rooms of a specialist physician or general practitioner.
2. The disclosure provides a method and an apparatus which significantly reduces the complexity associated with the use of conventional methods and is particularly useful in cases of urgent (e.g., burn) injury that have not been presented to the physician in time. In some instances, cells for grafting may be unavailable at the time of surgery, either due to delayed referral of a patient with an unhealed burn or simply because the time needed for culturing autografts had exceeded that for preparation of the recipient would bed. The present invention significantly accelerates cell preparation and ameliorates the issue of graft preparation time compared to CEA.
3. The subject invention aids in the achievement of rapid cell coverage in areas from donor to recipient sites. It provides a means for reducing the size of donor sites as the biopsy donor site required for the present invention is markedly smaller than a graft donor site required by conventional methods. As such, the present invention improves expansion ratio of cell coverage from donor to recipient sites; it also improves the rate of healing of acute wounds (such as burns and lacerations) and chronic wounds (such as venous or diabetic ulcers), is useful for skin reconstructions (such as scar removal or other aesthetic indications), and improves scar quality and/or minimizes scarring.
4. The subject invention ameliorates problems associated with the use of media or solutions used during conventional tissue culture process which require serum xenogenic to the patient. According to the method of preparation and treatment of the present disclosure, the cells can be in the form of a cell suspension, in a nutrient solution that is free of serum xenogenic to the patient. Such cell suspension can then be applied, dispersed or placed directly onto the recipient site, reducing or eliminating chances of infections, viral transmissions or other complications associated with xenogenic serum.
5. The disclosure provides a means for the treatment of various skin abnormalities, disorders or diseases. For example, it may be used for epidermal resurfacing, replacement after skin loss, site match-up during re-pigmentation of an area of skin, treatment of burn wounds, treatment of leukoderma, treatment of vitiligo, treatment of piebaldism, treatment of scars (caused by, for example, incorrect wound healing, improper scar direction or scar distortion from wound contraction), treatment of acne scars, resurfacing cosmetic dermabrasion, improvement of aesthetic indications such as in plastic or reconstructive procedures, resurfacing after laser treatment and in association with dermal reconstruction, treatment of artificial "wound" where the skin was intact but a "wound" is created (e.g., via laser or dermal burn or dermabrasion, or on a graft donor site), accelerating wound healing, reducing infections, and/or minimizing scarring. Additionally the method, cell suspension and composition of the present invention may be used for cell replacement therapy, including, for example, nerve cell replacement treatment, epithelial cell (such as urothelial cell, buccal mucosal cell and respiratory epithelial cell) replacement treatment, endothelial cell replacement treatment and osteogenic precursor cell replacement treatment. Furthermore, the present invention can be applied to any epithelium-related procedure, including skin epithelium and non-skin epithelium such as glabrous epithelium, respiratory epithelium, vascular epithelium, glandular epithelium, or corneal epithelium. That is, while some embodiments are described in connection with a skin-related procedure, the same can be applied to non-skin epithelial tissues with slight modifications that are well within the level of ordinary skills in the art.
6. The subject invention provides a means to produce a suspension of cells of several different types, wherein the amount of each type of cells is in a ratio to one another that is comparable with, or substantially the same as, the ratio of the cell types detectable in situ at the patient's donor and/or recipient site. That is, due to the unique manner of preparation of the cellular suspension, the relative percentage of different cell types (in the case of skin tissue, such as keratinocytes, Langerhans cells, fibroblasts and melanocytes) typically have the same percentage as at the donor site where the cells are harvested (in the case of skin tissue, near the dermal-epidermal junction). The cells so prepared also have enhanced survival rates in comparison to standard tissue culture techniques, where standard selective cell culturing can result in the loss of certain cell types while other cell types outgrow them. This provides, for example, the advantage of allowing for the correct re-surfacing or re-pigmentation of skin after a skin graft or other treatment or procedure.
7. The subject invention allows for faster surgery and healing, thereby reducing trauma for patients during medical care.
8. The subject invention provides, in the case of skin tissue, a suspension of skin stem cells a. Because skin stem cells are generally found in the basal layer of the epidermis and at the base of hair follicles, the present invention allows for the targeted harvesting of these cells from near the dermal-epidermal junction and from the dermal and/or epidermal layers of the skin. Skin stem cells can include epidermal stem cells and follicular stem cells. Typically the epidermal stem cells are responsible for regeneration of the different layers of the epidermis. Melanocyte stem cells give rise to melanocytes, and the follicular stem cells can give rise to both the hair follicle and to the epidermis. In addition, the base of hair follicles is also rich source of melanocytes that can be useful for wound healing and can be included in the cell suspension of the present invention.

### Preparation of Cell Suspension

According to an aspect of the invention there is provided a method for preparing a cell suspension suitable for use in the treatment, healing, reconstructing, resurfacing and/or regeneration of epithelial tissues.

Skin tissue (preferably of an autologous nature to the recipient patient) can be harvested from a patient by means known in the art of tissue grafting or regeneration. This can be achieved by taking a full-thickness or split-thickness tissue biopsy from a donor site of the patient. With the harvesting of the biopsy consideration includes the depth of the biopsy and the surface area size. The depth and size of the biopsy can affect the ease at which the procedure can be undertaken and/or speed with which the patient recovers from the procedure. The donor site may be chosen to match the recipient site, for example, post-auricular tissue for head and neck, thigh for lower limbs, inner-upper arm for upper limbs, or palm for sole or vice-versa.

The biopsy or tissue sample harvested from the donor site can then be subjected to physical and/or chemical dissociating means capable of dissociating cellular stratum in the tissue sample, thereby breaking the tissue sample into smaller pieces, or at least making different layers (e.g., dermis and epidermis) of the tissue sample more easily separable.

Various methods for dissociating cellular layers within the tissues known in the art can be used. For example, the dissociating means may be either a physical or a chemical means, or a combination of both. Physical dissociating means can include, for example, scraping the tissue sample with a scalpel, mincing the tissue, physically cutting the layers apart, using a pestle or mesh mortar or grater to dissociate, and/or perfusing the tissue. Chemical dissociating means can include, for example, digestion with enzymes such as trypsin, dispase, collagenase, trypsin-EDTA, thermolysin, pronase, hyaluronidase, elastase, papain and pancreatin. Non-enzymatic solutions for the dissociation of tissue can also be used.

In certain embodiments, dissociation of the tissue sample can be achieved by placing the tissue sample in an enzyme solution containing an amount of enzyme sufficient to dissociate cellular stratum in the tissue sample. This may be achieved, for example, using a trypsin solution. Other enzymes or proteases can also be used, such as dispase, collagenase, trypsin-EDTA, thermolysin, pronase, hyaluronidase, pancreatin, elastase and papain. Not wishing to be bound by theory, such enzymes are believed to cause cells to become detached from other cells or from solid surfaces via proteolysis of extracellular proteins. When the enzyme used is trypsin, the enzyme solution may be calcium and magnesium free. One such solution, for example, is calcium and magnesium ion free phosphate buffered saline.

Where the tissue sample is derived from a patient's skin (comprising both epidermis and dermis) the amount of trypsin or other enzyme used in the method can be between about 0.1% and 5% trypsin or other enzyme per volume of the solution. In certain embodiments, the trypsin or other enzyme concentration can be from about 0.25% to 2.5%, or at about 0.5%. Those of ordinary skill in the art will understand that experimenting the optimal concentration of the enzyme is routine experimentation.

The amount of time for which the tissue sample is subjected to the enzyme solution may vary depending on the size of the sample (e.g., thickness and surface area). Typically, the larger the size of the sample, the longer the sample should be subjected to the enzyme solution. In some embodiments, the tissue sample can be placed in the solution for a sufficient time to weaken the bonding or linkage between the tissue layers (e.g., between the epidermis and dermis). For example, where the tissue sample is taken from a patient's skin the sample might be placed in the enzyme solution from a few seconds to a few hours, or for a 5 to 60 minute period. Preferably, the tissue sample is immersed in the enzyme solution for between 10 and 30 minutes or 15 to 20 minutes. Those of ordinary skill in the art will understand that experimenting the optimal time of enzyme treatment is routine experimentation.

After the tissue sample has been immersed in the trypsin solution (or other enzyme solution) for a sufficient time, the sample can be removed from the solution and washed with a nutrient solution. Washing the tissue sample may involve either partial or complete immersion of the enzyme-treated sample in the nutrient solution. Alternatively, the wash solution can be dripped or dispersed onto the tissue sample in a sufficient volume to remove and or significantly dilute any excess enzyme solution from the tissue sample. In certain embodiments, after dilution or washing, no more than 0.05% trypsin or other enzyme is left.

The nutrient solution can significantly reduce or remove the effect of the enzyme by dilution and/or neutralization. The nutrient solution can be: (i) free of serum xenogenic to the patient, (ii) capable of maintaining the viability of the harvested cells, and (iii) suitable for direct application to a recipient site on the patient. The nutrient solution may be anything from a basic salt solution to a more complex nutrient solution. In some embodiments, the nutrient solution can contain various salts that resemble those found in body fluids, such as a physiological saline. The nutrient solution may be free of all serum. Phosphate or other non-toxic substances may also buffer the solution in order to maintain the pH at approximately physiological levels. One suitable nutrient solution is Hartmann's solution.

After washing the tissue sample with the nutrient solution, the tissue sample can be gently scraped permitting viable and functioning cells to be harvested from the tissue sample. The cells can include stem cells capable of reproduction (e.g., dividing or differentiation), as well as differentiated non-stem cells that perform specific functions (e.g., melanocytes function to produce melanin). An advantage of the present invention is the inclusion of non-stem cells that are functioning, which can help to promote treatment, healing, reconstructing, resurfacing, repigmentation and/or regeneration of epithelial tissues. Where the tissue sample is skin, the epidermis layer can be scraped first to expose the dermal-epidermal junction where lower surface of the epidermis and the upper surface of the dermis meet; then cells in the dermal-epidermal junction and the dermal layer can also be scraped. The so harvested cells include both skin stem cells and non-stem cells.

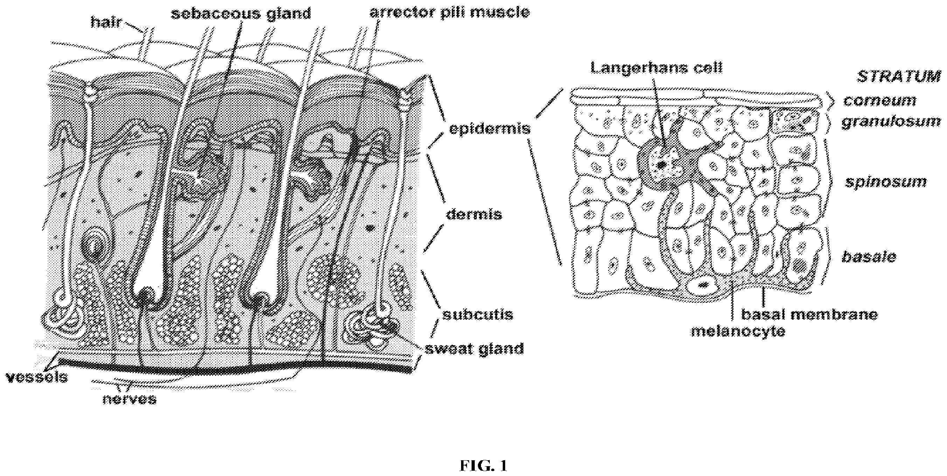

As shown in FIG. 1, skin epidermis contains keratinocytes, melanocytes, Langerhans' cells and Merkel cells. The epidermis forms the surface of the skin. It is made up of several layers of cells called keratinocytes. Keratinocytes in the basal or germinative layer or stratum (also known as keratinocyte basal cells or basal keratinocytes) undergo mitosis and proliferate. Keratinocytes undergoing differentiation progressively move to stratum spinosum, where they become polyhedral in shape and begin to produce tonofilaments. Keratinocytes are the most abundant cell type in the epidermis. Melanocytes locate in the basal layer of epidermis and represent every fifth to every tenth cell therein. Melanocytes produce the pigment melanin, and therefore play an important role in skin and hair follicle pigmentation. The Langerhans' cells reside suprabasally, and represent about 1% of epidermal cells.

Still referring to FIG. 1, the dermis lies underneath the epidermis and contains skin appendages: hair follicles, sebaceous (oil) glands and sweat glands. Cell types in the dermis include dermal fibroblasts which produce intercellular supportive matrix (e.g. collagens and elastin) and may have a role in wound healing, as well as mast cells, macrophages, melanocytes, Merkel cells, and cells belonging to the immune system and skin appendages.

Cells capable of reproduction can include skin stem cells and/or fibroblasts. Skin stem cells can include epidermal stem cells, melanocyte stem cells and follicular stem cells. Epidermal stem cells are found in the basal layer of the epidermis and are responsible for everyday regeneration of the different layers of the epidermis. Melanocyte stem cells are responsible for regeneration of melanocytes. Follicular stem cells ensure constant renewal of the hair follicles and can also regenerate the epidermis and sebaceous glands if these tissues are damaged. Hair follicle stem cells are found throughout the hair follicles.

Cells harvested from the entire skin sample including epidermis, dermis and dermal-epidermal junction thus can include skin stem cells and/or fibroblasts. In certain embodiments, the harvested cells can include keratinocytes, melanocyte stem cells, melanocytes, fibroblasts, and/or Langerhans cells. These cells can be harvested from the tissue sample by any suitable means known in the art. For example, the cells can be scraped off the surface of the sample, beginning from the epidermis until substantially all cells in the sample are collected, using an instrument such as a scalpel. The cells can also be disaggregated by being forced through a screen of appropriate size mesh. In one embodiment, cells so harvested include keratinocytes, Langerhans cells, fibroblasts and melanocytes. Following harvesting of the cells from the tissue sample they are present in the nutrient solution in a suspended form (e.g., dispersed).

The cellular suspension can then be filtered. Filtering can be used to remove or collect excessively large cellular congregates, so as to prevent clogging of the applicator and facilitate an even distribution of the cellular suspension to the recipient site. Any filter means capable of separating excessively large cellular congregates from the suspension may be used in this preferred step of the invention. In an embodiment, the filter size can be between 50 µm and 200 µm, between 75 µm and 150 µm, or at about 100 µm.

Prior to application/dispersion to the recipient site or immediately after filtering, the cellular suspension may be diluted to produce an appropriate cell density suitable for the purpose to which the suspension is to be used.

### Use of Cell Suspension

According to another aspect of the invention there is provided an aqueous cell suspension produced by the method described herein. The cell suspension provided by this method is suitable for use in various epithelium-related procedures. An important advantage of utilizing such a suspension in epithelium-related procedures is the possibility to greatly expand the area or size or volume of a wound or recipient site that can be treated quickly by in situ multiplication of a limited number of cells capable of reproduction. Where skin is involved, the epithelium-related procedure can be skin wound treatment, skin tissue graft, skin aesthetic procedure, or other epidermal and/or dermal treatment. Application of the cell suspension uniformly over the wound mimics the healing capacity of pre-natal skin, such that the wound can heal uniformly and close swiftly with full coverage by the cell suspension, resulting in optimized structure and function. The skin wound can be acute wound (e.g., burn or laceration caused by trauma), artificially created wound (e.g., in a graft donor site, aesthetic indication, plastic procedure or dermal treatment), or chronic wound (e.g., venous or diabetic ulcers). Other suitable indications include scar remodeling, glabrous skin loss injuries, pigmentation issues, vitiligo, leucoderma and cosmetic rejuvenation procedures. In various examples, the number and/or concentration of cells seeded onto recipient site may be varied depending on the procedure and/or recipient site, e.g., by modifying the concentration of cells in the suspension, and/or by using an appropriate quantity of suspensions that are distributed onto a given area or volume of the recipient site.

By placing cells in a nutrient solution which is at least (i) free of serum xenogenic to the patient, (ii) capable of maintaining the viability of the harvested cells, and (iii) suitable for direct application to a recipient site on the patient, it has been found that the outcome of patient treatment is improved. Not wishing to be bound by theory, a partial explanation may be attributable to the exclusion of xenogenic serum and in certain embodiments, any serum from the cell suspension. Xenogenic serum is a common additive in standard grafting culture medium and has been suspected to cause potential infective and hypersensitivity problems. Such serum is however generally required for the standard in vitro expansion of the cells and to neutralize the action of trypsin. The nutrient solution used in the present invention does not require such serum because the cell population within the suspension do not require expansion or multiplication prior to application to the recipient site. Rather, cellular multiplication occurs on the recipient site as opposed to in an in vitro culturing system. While in vitro culturing is desired, which is not according to the invention, a serum-free medium can also be used, optionally supplemented with suitable growth factors, agents such as hyaluronic acid and/or recombinant proteins such as heat shock proteins and angiopoietins.

In addition, the nutrient solution may contain exogenous agent(s) or component(s) such as heat shock protein(s), hyaluronic acid, platelet-enriched plasma, growth factor(s), cytokine(s), and/or adipose stem cells, such that the final composition for dispersion to the recipient site can contain such agent(s). Alternatively, such agents can be separately supplied to the recipient site. One or more of these agents can be supplemented to an in vitro culturing system (in a cell culture or where the cells are seeded to a biological or synthetic extracellular matrix or substrate). Heat shock proteins and hyaluronic acid are described in more detail herein. Platelet-enriched plasma (also referred to as PRP) is blood plasma that has been enriched with platelets obtained by removing red blood cell from peripheral blood by centrifugal separation at a low speed. PRP contains a large amount of growth factors such as platelet-derived growth factor (PDGF) contained in platelet, transforming growth factor β (TGF-β), fibroblast growth factor (FGF), or insulin-like growth factor (IGF), and has effects such as angiogenesis, osteogenesis, promotion of wound healing, or tissue regeneration by a synergistic action of these factors. Useful growth factors and cytokines include epidermal growth factor, transforming growth factor-a and -β, hepatocyte growth factor, vascular endothelial growth factor, platelet derived growth factor, fibroblast growth factor 1 and 2, insulin-like growth factor 1 and 2, interleukin 8, connective tissue growth factor, and keratinocyte growth factor. Adipose stem cells include various stem cells having proliferative and differentiating potentials collected from adipose tissues. Adipose stem cells can undergo adipogenic, osteogenic, chondrogenic, neurogenic and/or myogenic differentiation *in vitro.* The exogenous agent(s) or component(s) can also be supplied in a separate step.

Another unique feature of the cell suspension produced according to the method of the present invention is that the composition of cells in the cellular suspension is comparable to, i.e., similar to or substantially the same as the in situ composition from the donor site (e.g., adjacent the dermal-epidermal junction in the case of skin). One possible explanation is that in standard techniques, culturing of the skin cellular preparation utilizes selective culturing for keratinocytes, resulting in the partial or complete loss of cellular constituents such as fibroblasts and melanocytes in the case of skin. By contrast, the cellular suspension produced according to the present invention has a cell composition, i.e., relative ratio or percentage of different cell types, similar to or substantially the same as the in situ cell composition. Another feature of the cellular suspension produced according to the present invention is that the cells have maintained a high viability as they experience minimal treatment steps and minimal harvesting time, and are harvested in a gentle nutrient solution.

There is provided a method of treatment of a patient in need of an epithelium-related procedure. By this method the cellular suspension produced according to the present invention can be applied directly, after harvesting and filtering, to a recipient site of the patient. The recipient site can be a site of acute wound (e.g., burn or laceration caused by trauma), artificially created wound (e.g., in a graft donor site, aesthetic indication, plastic procedure or dermal treatment), or chronic wound (e.g., venous or diabetic ulcers). The recipient site may be any prepared wound bed, including the traditional "donor region" in a standard graft procedure from which skin is taken from, the traditional "recipient region" requiring traditional skin grafting, as well as wound bed in patients who do not require a traditional skin graft. For example, relatively minor burns or wounds that do not require traditional graft surgery by current standard of care can be treated by the "cellular graft" procedure of the present disclosure. Such cellular graft can be applied as a stand-alone procedure, or in combination with or complementary to the traditional graft surgery. For instance, where a meshed graft tissue is used in a traditional graft, the cell suspension of the present invention can be applied to the meshed graft to improve epithelial regeneration.

The epithelium-related procedure can be treated by the cellular graft of the present invention includes without limitation, skin wound treatment, skin tissue graft, skin disease treatment (e.g., vitiligo or leucoderma, and chronic ulcers), skin aesthetic procedure (e.g., wrinkles and acne scars), or other epidermal and/or dermal treatment.

A liquid suspension containing cells of the present invention may be distributed onto the recipient site by any of several techniques, including without limitation, spraying, spreading, pipetting and/or painting. Also, the suspension may be applied to a wound dressing that is then applied to the wound, or introduced beneath an in-place dressing, or seeded onto a dressing before application. In one example, the cell suspension can be sprayed on to a recipient site. The suspension may be sprayed through any type of nozzle that transforms liquid into small airborne droplets. This example is subject to two considerations. First, the cells in the solution should not be subjected to excess shearing forces or pressures that may damage or kill a substantial number of cells. Second, the cellular suspension should not be mixed with a propellant fluid that is toxic or detrimental to the cells therein or the wound bed. A variety of nozzles that are commonly available are suitable for use. Such nozzles may be connected in any suitable way to a reservoir that contains the cellular suspension.

Alternatively the cell suspension may be delivered via a pipette, an eye-dropper like device, a syringe and needle, and/or other similar devices to place small quantities of the cell suspension on a recipient site. The cell suspension can also be injected into the tissue at the recipient site.

After the cell suspension has been applied to the recipient site, the site or wound bed may be dressed with a wound dressing. Various types of dressings can be used, including films (e.g., polyurethane films), hydrocolloids (hydrophilic colloidal particles bound to polyurethane foam), hydrogels (cross-linked polymers containing about at least 60% water), foams (hydrophilic or hydrophobic), calcium alginates (nonwoven composites of fibers from calcium alginate), hyaluronic acid derivatives (e.g., esters) and cellophane (cellulose with a plasticizer). See Kannon et al., Dermatol. Surg. 21:583-590 (1995); Davies, Burns 10:94 (1983). In an example the dressing can be Surfasoft™ which is a woven nylon dressing or Hyalomatrix® which is a hyaluronic acid ester matrix. In general, the healing of the wound bed is followed by standard protocols for skin graft treatment known to those skilled in the art.

The cell suspension of the present invention can also be applied to a tissue or organ regeneration system (in vivo or in vitro), such as extracellular matrix or matrix material (e.g., as in the re-growth of trachea). In various embodiments, the extracellular matrix or matrix material used can be synthetic or biological, such as collagen, alginate, alginate beads, agarose, fibrin, fibrin glue, fibrinogen, blood plasma fibrin beads, whole plasma or components thereof, laminins, fibronectins, proteoglycans, HSP, chitosan, heparin, and/or other synthetic polymer or polymer scaffolds and solid support materials, such as wound dressings, that could hold or adhere to cells. In certain embodiments, the cell suspension as applied to the regeneration system can facilitate the epithelial regeneration thereon. Upon completion, the regenerated system can be transplanted to a patient in need thereof.

### Device for Making and Using Cell Suspension

There is provided an apparatus for developing a tissue regeneration solution. The apparatus can have a heating means suitable for heating an enzyme solution to a predetermined temperature and capable of maintaining that solution at the desired temperature for a suitable amount of time. The apparatus can also include a filter means, which is capable of separating large cellular congregates (e.g., having a diameter larger than about 200 µm) from a cellular suspension.

The apparatus may also include a reservoir capable of holding a tissue sample immersed in a nutrient solution capable of maintaining the viability of the cells in the tissue sample. The reservoir may be of a sufficient size to permit manipulation of the tissue sample, for example, permitting separation of the tissue cellular stratum (e.g., epidermis and dermis) and harvesting cells from the epidermis and/or dermis, thereby producing a cell suspension for use in an epithelium-related procedure.

The apparatus may also include one or more fluid containment wells or pouches for storage of fluids such as the nutrient solution. The wells may alternatively serve as a receptacle for a container such as a plastic or glass vial that holds the nutrient solution. Preferably, the well is capable of holding at least a 10 ml volume. Such wells permit convenient application of the fluids to the tissue sample. Storage of such fluids in close proximity also provides the advantage of reducing the risk of accidental leakage of the fluids and allows easy access to the fluids for accurate delivery to either the tissue sample or the cell suspension.

The apparatus may include a first and second member wherein:
(1) the first member includes:
   (a) at least one heating means suitable for heating an enzyme solution to a predetermined temperature and capable of maintaining that solution at the desired temperature for a suitable amount of time;
   (b) at least one filter means capable of separating large cellular congregates from a cellular suspension; and
   (c) at least one fluid container for storage of a nutrient solution;
(2) the second member forms a reservoir capable of holding a tissue sample immersed in the nutrient solution; and
   wherein the first member provides a seat upon which the second member may be placed during manipulation of the tissue sample.

The first member may provide a storage compartment into which tools and solutions used in the methods described herein may be stored. Where such a compartment is provided in the apparatus, the second member may provide the lid or closure to that compartment. In use, the lid can be removed from the top of the compartment and can be inverted. The underside of the lid preferably forms the reservoir therein enabling the second member to serve a dual purpose. Tools and solutions used in the method can be accessed from the compartment. The inverted lid may then be seated back over the compartment therein providing the reservoir for the apparatus.

The apparatus may be made from metal, plastic or any other suitable material. Further, the container may be of any suitable size, depending on its intended use and the need for sterilization such as by gamma irradiation and/or ethylene oxide treatment.

It should be appreciated that the heating means employed in the apparatus may be a heating pad or pads on the top of the first member. To avoid accidental spillage of the container being heated, in an example, one or more heating means may be housed within a recess in the first member. Also located within that recess can be at least one container into which the tissue sample may be placed for exposure to the enzyme solution. In an alternate example one or more heating means may be housed in the base of the apparatus. In such a configuration the first member can contain at least one opening for receiving a container for holding a fluid, which opening can provide access for the container to the heating means.

It will be appreciated that if the apparatus is designed for multiple usages, the heating means may be repeatedly heated and cooled. Alternatively multiple heating units may be provided with the apparatus to facilitate multiple heating events, where each heating unit may be designed for single use.

In an exemplary configuration of the apparatus, a heating collar can be located within a recess therein, forming a heating recess in the first member within which a container (e.g., a vial) for the enzyme is located. The container can be held in place by at least one restraining means, which preferably surrounds at least part of the upper portion of the container, thereby preventing accidental release of the container from the apparatus. In certain examples, where the apparatus is intended for single use the restraining means may be formed as an integral part of the first member, thus the container may not be removed unless the first member is physically broken or damaged.

The heating means used in the apparatus can be controlled by circuitry permitting activation of the heating element when required. For example, the heating means may be switched on by depressing a start button located, for example, on the surface of the first member, or via a touchscreen user interface. Alternately the heating means may be activated by pushing the container down with sufficient force to activate a switch located in the base of the apparatus. A person of ordinary skill in the art will appreciate that a wide range of electronic means may be used to activate the heating unit provided in the apparatus.

The heating unit can also be operably linked to a timer mechanism, which can be adapted to heat the enzyme solution for a pre-defined period of time. In certain examples where the apparatus is intended for multiple uses, the timer can be set to deactivate the heating element when a predetermined amount of time lapses. Then an alarm may activate to inform the user that the time is up. The alarm may be audible or in the form of a light display.

In a further preferred example, the heating means may be provided with an adjustable temperature control. Where temperature adjustment is required, such adjustment may be achieved by adapting the heating control circuitry to include or communicate with a temperature control mechanism permitting the temperature of the heating unit to be constantly varied within a constant range, or it may present a range of select temperatures that the heating control means can be set at. A temperature control means can be included in the apparatus where the apparatus is to be used in the harvesting and preparation of different cell types and/or where different enzymes are used in the harvesting method for which the apparatus has unique application.

In some examples, the apparatus can be designed for single use. In such instances the timer mechanism can be part of the circuitry that controls the heating means. Once the heating means has been activated, it heats the solution for a predefined period of time and then self-destructs. It should be appreciated by those skilled in the art that such an the apparatus may be fitted with various monitoring means that are capable of indicating one or more of: the enzyme solution has reached the predetermined temperature; the amount of time that the enzyme has been in the solution for; and/or the amount of time left before the circuitry self-destructs. By way of example only, the monitoring means might consist of a series of LED's that activate when certain events occur. In an example, the heating element remains in the heating mode for a maximum of 45 minutes to 1 hour.

The heating means may be powered by any means known in the art. Preferably, the power supply is provided by battery/batteries. In one example, the power supply is a battery or a plurality of batteries located in the base of the apparatus.

In a further example, the apparatus may be provided with one or more means to facilitate mixing of the solutions used in the invention, for example, an enzyme solution. In this respect, and by way of example only, the apparatus may include a means for vortexing the solution, such as an electromagnetic system that is adapted to agitate a magnetic bead. Where the apparatus includes an electromagnetic mixing system, the magnetic bead can be provided in the container (e.g., a vial) in which the solution is stored in the apparatus. Alternatively the magnetic bead may be added to the solution when mixing is desired.

In an example, the mixing means can be combined with the heating means either as a single unit or as separate units to facilitate constant heating of the solution in an even manner. Using such a mixing means can avoid overheating of the solution closest to the heating unit while the solution is heated. Such a system can provide a constant heating of the solution. Alternate means for mixing the solution are known in the art and include, for example, mechanical, physical, electrical and electromagnetic means. While any mixing means may be employed in the apparatus, preferably the mixing means is either selected to minimize vibration of the apparatus or incorporated into the apparatus in a manner that minimize such vibration. In this respect the mixing means may be housed on one or more vibration dampeners or the apparatus may include one or more vibration dampeners on its base.

Where a mixing means is incorporated into the apparatus, the means may be automatically activated upon activation of the heating unit or alternatively there may be a separate activation system. Further, the speed of mixing may be fixed or variable. Preferably, there is a separate activation system for the mixing means.

There can be a filter recess incorporated into the apparatus may be of any size or shape that facilitates filtering of a cellular suspension to remove or collect large cellular congregates (e.g., larger than 200 µm, larger than 150 µm, or large than 100 µm). Further the recess may be adapted to receive and hold at least one tube or vial into which cell suspension may be filtered. The recess can have a conical base providing easy access the full volume of the cell suspension after filtering. The filter means can also be an in-line filter.

In some examples, a recess is designed to receive a 100 µm cell filter that is capable of removing or collecting cellular congregates larger than about 100 µm. The use of the filter can also prevent the sprayer/nozzle from clogging by the large congregates. The recess can accommodate a 100 µm cell filter connected to a conical tube. Preferably, the tube has area/volume graduations marked on the side.

The apparatus may also include a set of tools required for cell harvesting. It will be appreciated by those skilled in the art that any tools necessary for cell harvesting may be included with the device. Preferably, the set of tools are sterile. As an example, the set of tools may include a glass vessel of separation enzyme; a sterile solution for suspension of the enzyme; a sterile nutrient solution; scalpel; forceps; syringe; medicine dropper, cell filter; wound dressings and/or spray nozzles. In one example, the set of tools are stored in a compartment formed in the first member of the apparatus, which is covered by the second member when not in use.

In an example, the apparatus is used to harvest and prepare a suspension of cells, and further to apply the suspension of cells to a recipient site in any suitable manner.

For example, an aliquot of sterile water can be mixed with a portion of lyophilized separation enzyme and placed in the heating recess. The heating means is then activated which heats the contents (i.e., the enzyme solution) of the container to a working temperature of between about 30 and 37 °C, preferably between about 33 and 37 °C, or by way of example about 37 °C within a short time (e.g., about 5 minutes, about 3 minutes, about 2 minutes, or about 1 minute), and maintains the working temperature for a sufficient time to allow enzyme digestion to proceed (e.g., about 30 minutes, about 45 minutes, about 0 minutes, or longer or shorter). Once an operational temperature has been reached, a sample of tissue taken from a donor site is placed in the enzyme solution and incubated at the working temperature. The tissue sample is incubated for a sufficient time (e.g., between 5 to 60 minutes, between 10 to 45 minutes, between 20 to 30 minutes, or longer or shorter). Those skilled in the art would appreciate that the time taken to achieve separation of the layers of the tissue sample is dependent on the thickness and size of the tissue sample and the incubation temperature, and can be determined via routine experimentation. Once enzymatic separation of the tissue layers is achieved, the tissue sample is removed, rinsed with a nutrient solution, and placed into the reservoir where the tissue layers are held in place using surgical instruments such as forceps.

A carefully measured aliquot of the second solution (e.g., a nutrient solution) is then withdrawn from the fluid containment well by aspiration into a syringe and then applied to the layers. The cells from the entire tissue sample (e.g., including epidermis, dermis and the dermal-epidermal junction) are scraped off using a scalpel, and become suspended by mixing with the nutrient solution. The cell suspension is then collected, preferably using a syringe and cannula.

The harvested suspension of cells is then passed through a cell filter located in the filter recess to move large cellular congregates and other unwanted materials, and the filtered suspension of cells is collected into the filter recess. The reservoir may optionally be rinsed with a further volume of the second solution and this resulting further suspension of cells can also be filtered and collected in the filter recess.

The filtered suspension of cells may then optionally be aspirated into a dispersing means (e.g., syringe) and applied directly to the recipient site. The suspension can also be applied indirectly to area in need of repair, e.g., a scaffold/substrate or dressing, either in vitro or in situ.

In further embodiments, the process of preparation of the suspension can be automated. An automated device can include a processing unit capable of multiple uses along with consumables for single use. For example, an automated device having disposable cartridges can be provided. The disposable cartridges can have blisters containing the water, enzyme and buffer. Rollers or pressure plates can be used to create pressure to burst one-way valves, introducing the contents of the blisters into a processing chamber containing the biopsy. After enzyme incubation, the biopsy can be processed in a fashion similar to mortar and pestle, where the base can contain a mesh or any other scraping device to complete the scraping step. In some examples, the device can have more than one disposable cartridge, and therefore can process more than one biopsy in parallel.

### Use of Heat Shock Protein

Heat Shock Proteins (HSPs) were initially discovered as proteins that were markedly induced by heat shock and other chemical (e.g., compounds) or physical stresses. Since that time they have been viewed as molecular chaperones, guiding and modifying the structures of proteins by their intrinsic ability to re-fold them from a denatured to a native structure. These interactions and the balance toward fully-natured functional proteins necessitates that the HSPs be abundant. The HSPs have been generally classified into several classes depending on their molecular weights. For example, the Hsp27, 40, 70 and 110 genes have evolved to be particularly efficient for mass synthesis after or during stress with powerful transcriptional activation, efficient messenger RNA (mRNA) stabilization, and selective mRNA translation. Hsp27, 70, 90, and 110 levels increase to become the dominantly expressed proteins after stress.

Heat shock proteins may play an important role in the protection of the skin from environmental stresses and participate in the prevention and repair of damages caused by exposure to light, heat, chemical injuries, and other traumas. In addition, UV-B irradiation can also induce heat shock response, which protects human skin from cell damage and is part of the natural defenses that follow exposure to solar radiation. However, in physiological wound healing, few cells are stressed at any particular time, and therefore, most cells are not activated, or secreting HSPs or proliferating.

Therefore, wound healing processes may benefit from the exogenous induction of a heat shock response. For example, by placing the cell suspension of the present disclosure under stress conditions, the cells can exhibit a heat shock response. Suitable stress conditions include without limitation, hypoxia, heat shock, change in pH, change in osmotic and/or atmospheric pressure, chemical compounds, radiation, mechanical trauma, nutrient deprivation, and/or fasting or calorie restriction. Hypoxia can be achieved by incubating the cells in an environment lacking adequate oxygen supply. In addition, as the method of the present disclosure takes the biopsy away from its normal vascular supply, the cells in the biopsy, as well as the cells harvested from the biopsy, can experience stress and express stressed and/or activated state due to the reduced or absent vascular supply. The process of heat shocking can be done in a CO₂ incubator, O₂ incubator, or a hot water bath. Furthermore, stress can be induced throughout the procedure of preparing and/or dispersing the cell suspension, for example, from removing the biopsy from the vascular supply, from disaggregating the biopsy into a suspension of cells (e.g., via enzymatic and/or mechanical means), and/or from aerosolizing or otherwise dispersing the cells. Under stress, the cells can display a stress-induced characteristic, for example, an elevated level of expression of a heat shock protein. The heat shock protein can be Hsp90 and/or Hsp90α.

Hsp90 is one of a number of classes of HSPs, which are generally synthesized in response to cellular stress as well as being constitutively expressed in some cases. These types of stress are numerous, but include heat shock, metal toxicity, nutrient deprivation, oxidative stress as well as numerous disease states. Hsp90 is one of the most prevalent chaperone proteins present in the cell - it can represent 1-2% of unstressed cell's protein and as much as 6% in the stressed cell. It has the ability to provide multi-component complexes which have been shown to include p60/Hop, p50Cdc37, Hsp40/HDJ2, p23, Hsp70 and one of a number of immunophilins (such as FKBP51 and FKBP52).

Hsp90 is generally present in two forms, Hsp90α and β. Both proteins are highly related and appear to have identical activities; however, the former is inducible whereas the latter is constitutively expressed. Hsp90 is also a phospho-protein and has two or tree bound phosphate molecules per monomer and exists as a homodimer. One of the clear distinctions that have made it a potential drug target are the fact that a significant amount of its client proteins are protein kinases. These client proteins include ErbB2/Her-2, EGFR, Hif1α, c-Met, Akt/PKB, Raf-1, Cdk-1 and 4, Aurora B, Ask1, CHK1, CKII as well as mutant p53. Furthermore, due to the nature of the interactive and complex network that is associated with regulating cell proliferation and cell death, the approach of identifying one specific (drug) target and blocking or activating it can result in the cell simply altering its regulation in order to maintain the phenotype.

Recently, HSPs were found to be actively secreted by cells and carry out important extracellular functions, including stimulation of immunological cytokine production, activation of antigen presenting cells (APCs) and anti-cancer functions. Hypoxia causes Hsp90α secretion in both epidermal and dermal cells. The secreted Hsp90α in turn promotes migration of these cells. Since HSP proteins lack any signal sequences at the amino terminus, these proteins cannot be secreted via the classical endoplasmic reticulum/Golgi transport pathway. Instead, these proteins are secreted to outside of the cells by a discrete population of nano-vesicles (30-90 nm in diameter), called exosomes. Therefore, the exosome secretion constitutes a potential mode of intercellular communication and opens up new therapeutic and diagnostic strategies. TGFα "pushes" Hsp90α out of the human keratinocytes via the exosome pathway, which in turn promotes migration of both the epidermal and dermal cells through the cell surface receptor CD91/LRP-1 ("LRP" meaning LDL receptor-related protein-1).

Accordingly, in certain embodiments, the cell suspension of the present invention, or a portion thereof, can be co-administered to a recipient site with a heat shock protein, or a fragment thereof, to facilitate wound healing or otherwise promote treatment, regeneration, resurfacing of epithelial tissues in an epithelium-related procedure. The cell suspension and the HSP can be supplied in a mixture and dispersed to the recipient site. Alternatively, the cell suspension and the HSP can be applied to the recipient site separately. Multiple applications or dispersions of the cell suspension and/or the HSP can also be used to achieve the desirable wound healing outcome. The HSP or a fragment thereof can be in an effective amount to promote wound healing. The HSP can be Hsp90 and/or Hsp90α. The HSP or fragment thereof can be formulated in a concentration of from about 0.1 µg/µl to about 100 µg/µl, from about 0.3 µg/µl to about 50 µg/µl or from about 1 µg/µl to about 10 µg/µl.

U.S. Patent No. 8,022,037 discloses Hsp90α, specifically its middle domain plus the charged sequence, as an extracellular pro-motility factor for human epidermal keratinocyte (HKC), dermal fibroblast (DF) and microvascular endothelial cell (HDMEC) migration.

In various examples, HSP (e.g., Hsp90 and Hsp90α) or a fragment thereof, can be produced in large quantity and purified using conventional cloning and recombinant DNA technology. HSP can be supplied as a component of the apparatus of the present disclosure, e.g., in a dry powder format, or supplied in a liquid application or on a bandage. HSP can also be used as a stand alone treatment and provided in various formats; when provided in a liquid application, the HSP can be sprayed, dripped, dispersed, or otherwise applied to a recipient site; when provided on a bandage, the HSP can be formulated as a slow-release formulation using methods known in the art. It is also possible to use HSP in combination with a monoculture having a single type of cells, e.g., skin stem cells, fibroblasts, keratinocytes, etc., or with a mixture of cells thereof.

In various examples, HSP can help or augment wound healing and tissue regeneration by enhancing both the re-epithelialization process and recruitment of the cells. Without wishing to be bound by theory, the mechanism may be that "positively stressed" keratinocytes (e.g., both in a hypoxic fashion and via ReCell processing) can produce enhanced release of key proteins (e.g.,HSP90), platelets (releasing PDGF), and stem cells (releasing proliferators, e.g., VEGF), cytokines (immunomodulators) and chemokines (migrators) that can regulate and control the process of regeneration; HSP90 can block action of TGFb, disinhibiting response to VEGF and PDGF by endothelial cells and fibroblasts resulting in proliferation and differentiation of key cell types accompanied by rapid development of blood vessels and nerves. The interplay of inhibition, disinhibition and activation generates a positive feedback cascade resulting in further release of growth factors, cytokines and chemokines.

### Use of Hyaluronic Acid

The term "hyaluronic acid" as used herein includes alkali metal salts such as sodium, potassium and lithium salts of hyaluronic acid. The term "hyaluronic acid" is also intended to include not only elemental hyaluronic acid, but hyaluronic acid with other trace of elements or in various compositions with other elements, as long as the chemical and physical properties of hyaluronic acid remain unchanged. In addition, the term "hyaluronic acid" as used in the present application is intended to include natural formulas, synthetic formulas or combination of these natural and synthetic formulas.

Hyaluronic acid (also called hyaluronan or hyaluronate or HA) is a glycosaminoglycan distributed widely throughout connective, epithelial, and neural tissues. More specifically, HA is a linear, unbranched polysaccharide made of alternating *N*-acetyl-D-glucosamine and D-glucuronic acid and can reach chain lengths of up to 20,000 disaccharide units or higher. One of the chief components of the extracellular matrix, HA contributes significantly to cell proliferation and migration. The presence of HA in epithelial tissue has been shown to promote keratinocyte proliferation and to increase the presence of retinoic acid, effecting skin hydration. Hyaluronic acid's interaction with CD44 drives collagen synthesis and normal skin function. Present predominantly in the extracellular matrix of basal keratinocytes, HA is critical to the structural integrity of the dermal collagen matrix.

In normal skin, HA is found in relative high concentrations in the basal layer of the epidermis where proliferating keratinocytes are found. CD44 is collocated with HA in the basal layer of epidermis where it has been shown to be preferentially expressed on plasma membrane facing the HA-rich matrix pouches. Maintaining the extracellular space and providing an open and hydrated structure for the passage of nutrients are the main functions of HA in epidermis.

HA can also affect or promote wound healing. Skin wound healing is a complex process, and includes many interacting processes initiated by haemostasis and the release of platelet-derived factors. The following stages include inflammation, granulation tissue formation, re-epithelization and remodeling. HA can play a multifaceted role in mediation of these cellular and matrix events. First, in the inflammation process, many biological factors, such as growth factors, cytokines, eicosanoids etc., are generated. These factors are necessary for the subsequent steps of wound healing due to their roles in promoting migration of inflammatory cells, fibroblasts, and endothelial cells into the wound site. The wound tissue in the early inflammatory phase of wound repair contains abundant HA, probably a reflection of increased HA biosynthesis. HA can act as a promoter of early inflammation, which is crucial in the whole skin wound-healing process.

Granulation tissue is the perfused, fibrous connective tissue that replaces a fibrin clot in healing wounds. HA is abundant in granulation tissue matrix. A variety of cell functions that are essential for tissue and wound repair may attribute to this HA-rich network, including facilitation of cell migration into a temporary wound matrix, cell proliferation and organization of the granulation tissue matrix. For example, the HA-rich network or extracellular matrix can act as a conductive environment for migration of cells into the temporary wound matrix.

HA also functions in the re-epithelization process. It serves as an integral part of the extracellular matrix of basal keratinocytes, which are major constituents of the epidermis; its role in keratinocyte proliferation and migration are also important. In the wound healing process, HA is expressed in the wound margin, in the connective tissue matrix, and collocating with CD44 expression in migrating keratinocytes, where HA and CD44 act together to regulate keratinocyte proliferation and migration. HA may also act to reduce collagen deposition and therefore lead to reduced scarring.

Thus, in certain embodiments, the cell suspension for use according to the present invention, or a portion thereof, can be co-administered to a recipient site with HA (or a pharmaceutically acceptable salt thereof, such as sodium hyaluronate), to facilitate wound healing. The cell suspension and the HA can be supplied in a mixture and dispersed to the recipient site. Alternatively, the cell suspension and the HA can be applied to the recipient site separately. Multiple applications or dispersions of the cell suspension and/or the HA can also be used to achieve the desirable wound healing outcome. The HA can be provided in an effective amount to promote wound healing, such as from about 0.01 mg/mL to about 60 mg/mL, from about 1 mg/mL to about 50 mg/mL, from about 5 mg/mL to about 40 mg/mL, from about 10 mg/mL to about 30 mg/mL, or from about 20 mg/mL to about 25 mg/mL. For example, the amount of HA in compositions according to the invention can be 0.1, 1, 2, 4, 6, 8, 10, 20, 30, 40, 50, or 60 mg/mL. The HA can have any suitable molecular weight, ranging from a few kDa to millions of daltons, depending on the source, degradation, and purification procedure. The HA can be from about 0.01 to 9×10⁶ Da, about 0.5 to 9×10⁵ Da, or about 0.8 to 8×10⁵ Da, or higher or lower.

In various embodiments, HA of desirable molecular weight can be produced in large quantity and purified using conventional cloning and recombinant DNA technology (e.g., using hyaluronan synthase and/or other HA biosynthesis genes in recombinant cells). HA can also be obtained commercially from rooster combs, certain attenuated strains of group C. *Streptococcus* which synthesize this compound naturally as part of their outer capsule, or from bovine vitreous humor. In addition, molecular mass fractions of purified HA can be purchased from commercial sources including Fluka Chemical Corporation (Ronkonkoma, N.Y., USA), Genzyme Corporation (Cambridge, Mass., USA), Lifecore Inc. (Chaska, Minn., USA), Hyal Pharmaceutical Corporation (Mississauga, Ontario, Canada) and Bioniche Life Sciences, Inc. (Belleville, Ontario, Canada).

HA can be provided in various forms, such as solution, hydrogel, or as nanoparticles or microparticles. U.S. Patent Nos. 6,660,853 and 7,371,399 describe methods of making HA and a polymer gel containing HA.

HA can be supplied as a component of the apparatus of the present disclosure, e.g., in a dry powder format, or supplied in a liquid application or on a bandage. HA can also be used as a stand-alone treatment and provided in various formats; when provided in a liquid application, the HA can be sprayed, dripped, dispersed, or otherwise applied to a recipient site; when provided on a bandage, the HA can be formulated as a slow-release formulation using methods known in the art. It is also possible to use HA in combination with a monoculture having a single type of cells, e.g., skin stem cells, fibroblasts, keratinocytes, etc., or with a mixture of cells thereof.

### EXAMPLES

Further features of the present invention are described in the following Examples. It is to be understood, however, that these examples are included solely for the purposes of exemplifying the present invention.

The following examples are put forth so as to provide those of ordinary skill in the art with a exemplary description of how the compositions and/or methods claimed herein are made and evaluated, and are intended to be purely exemplary of the invention. Modifications and variations of various aspects of the following examples will be apparent to those skilled in the art. For example, while some examples are described in connection with a skin-related procedure, the same can be applied to non-skin epithelial tissues (such as respiratory epithelium, vascular epithelium, glandular epithelium, or corneal epithelium) with slight modifications that are well within the level of ordinary skills in the art.

### Example 1

### Preparation of Recipient Site

To optimize the success of the epithelium-related procedure, the wound was cleaned and assessed to be of the appropriate depth. Further, blood homeostasis was established and the wound was checked for evidence of surrounding cellulitis or infection. Techniques for preparing the area included, without limitation, sharp dissection, dermabrasion or laser-resurfacing.

### Donor Site Biopsy

The donor site was chosen to appropriately match the recipient site. The donor site was infiltrated with local anesthetic and adrenaline underneath the skin near the subcutaneous tissue. This allowed the donor site to be firm and aided in the taking a biopsy. The dimensions of the biopsy were determined by the size of the surface area of the recipient site to be covered. Typically, the present disclosure allows for a biopsy size that has an expansion ratio of about 1:10 to 1:80 (donor biopsy size: recipient site size). In one example, a biopsy size of 2 cm x 2 cm was taken from the donor site giving an expansion ratio of 1:60.

### Skin Resurfacing Using the ReCell® Technique

Treatment of the wound was carried out using the ReCell® Rapid Technique cell harvesting apparatus, which is explained in more detail in Example 2 below. The apparatus contained all the instruments, solutions, enzymes and dressings required for wound treatment.

The heating element was activated by depressing the "start button". Solution A (sterile water for injection) (10 ml) was transferred from the supplied plastic vessel marked Solution A into a glass vessel containing the separation enzyme (lyophilized trypsin) to give a final concentration of 0.5% trypsin. The enzyme solution was then mixed together, transferred to a vessel already located in the heating element recess and heated to about 37 °C.

The vessel containing Solution B (nutrient solution) was transferred from its supplied vessel into the fluid containment well.

The previously obtained tissue sample was then placed in the enzyme solution and incubated at about 37 °C for between 10 to 15 minutes. After incubation, the tissue sample was removed from enzyme solution with a pair of forceps, rinsed by dipping into the fluid containment well containing Solution B, and placed with the dermal side down and the epidermal side up in the reservoir.

Solution B was then aspirated from the well into a syringe and dripped from the syringe onto both layers of the biopsy.

The skin layers, dermis and epidermis were handled using forceps and scalpel. Beginning from the upper surface of the epidermis and followed by the zone of the dermal-epidermal junction where the lower surface of the epidermis and the upper surface of the dermis meet, cells are scraped off the sample until substantially all cells in the sample are collected, to develop a plume of cells in the reservoir. The cells were then mixed in Solution B by agitating and/or pipetting. The plume of cells was then drawn up into the syringe via a 19 gauge cannula.

The supplied filter (100 µm cell filter) was mounted in the filter recess and the plume of cells in Solution B was passed through the filter. A further small amount of Solution B was then used to rinse the reservoir (e.g., a petri dish) and collect any remaining cells, which were also passed through the filter.

The resulting suspension of cells collected in the conical recess was aspirated into a syringe and a nozzle was attached to the syringe for spraying or dripping on to the wound area.

The wound was re-checked to ensure that it was clean and free of debris and that there was no evidence of bacterial contamination. Further, the wound was checked to determine if homeostasis had been achieved. Once the recipient site was ready, the suspension of cells was applied to the wound surface using the nozzle.

The wound was dressed with Surfasoft™, a woven nylon dressing, which was supplied with the apparatus. The healing of the wound was followed up using standard protocols for skin-graft treatment.

### Example 2

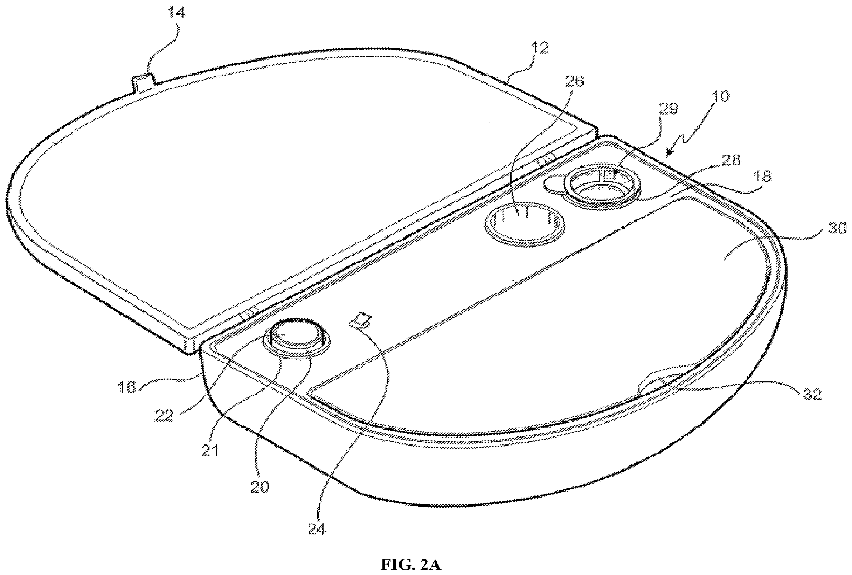

The example shown in FIG. 2A is directed to a ReCell® Rapid Technique cell harvesting apparatus 10 for use in producing a transplantable cellular suspension of living tissue suitable for applying to a patient in an epithelium-related procedure.

As illustrated in FIG. 2A the apparatus includes a closure lid 12 possessing a locking mechanism 14 adapted to releasably engage a base portion 16. The locking mechanism 14 provides a means for closing the apparatus 16 when not in use. Located within the base portion 16 is a first member 18 within which there is provided an aperture 20 in which there is located a vial 22 for the enzyme. Adjacent the aperture there is provided an activation switch 24 capable of activating the heating means (not shown). The first member also provides a fluid containment well 26 and a filter recess 28. As presented in this illustration the filter 29 is shown located in the filter recess. Ordinarily the filter is an optional item included as a separate item in the apparatus.

The aperture 20 in the first member 18 is desirably of such a diameter that it allows the neck of the vial 22 to protrude through and above the first member 18. The periphery of the aperture 20 is fitted with a collar 21 which is slightly smaller than the diameter of the body of the vial 22. Thus, when in use, the vial 22 cannot be removed from the apparatus 10 as it is held in place by the collar 21 located around the periphery of the aperture 20.

Located adjacent to the aperture 20, fluid containment well 26 and filter recess 28 is the second member 30 which is positioned on a seat (not shown) located within a storage compartment (not shown) within the first member 18. When inverted the second member 30 forms a reservoir within which tissue manipulations may be performed. To facilitate separation of the second member 30 from the first member 18 an indent 32 is provided in the side of a portion of the second member 30, which is of such a size that a person can lift the second member from the seat on which it resides in the first member 18.

Within the filter recess 28 there is located a filter 29 (provided separately with the other components) having a mesh therein capable of separating cellular material of greater than 100 µm from a cell supernatant.

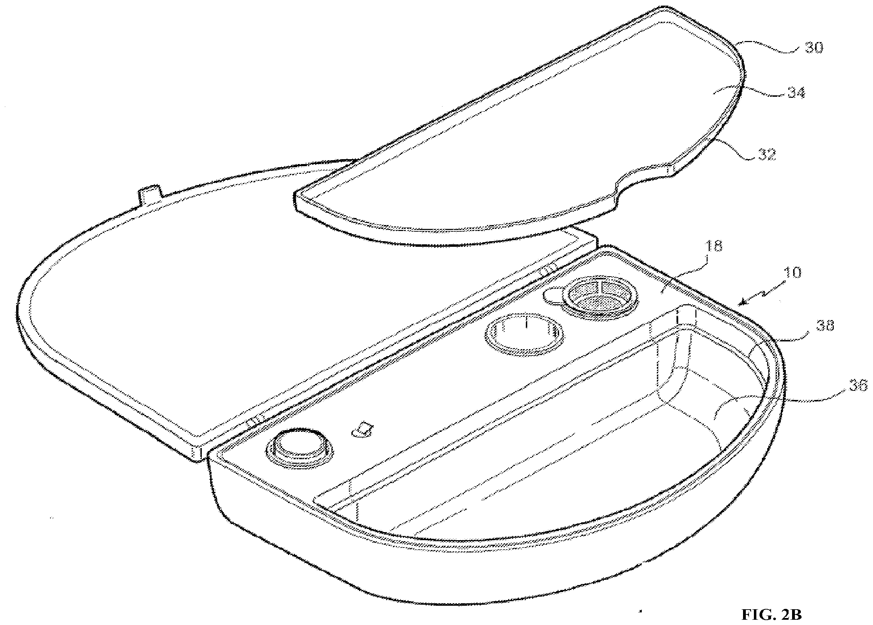

FIG. 2B provides a partially exploded perspective view of the apparatus 10 wherein the second member 30 is removed from the first member 18 and inverted. Inversion of the second member 30 reveals the sidewalls 32 of the second member 30, which form the fluid containment barrier of the reservoir and a reservoir area 34 in which tissue manipulations may be performed.

Removal of the second member 30 from the first member 18 also reveals a storage compartment 36 in the first member 18 in which solutions and tools may be stored when the apparatus 10 is not in use. Within the storage compartment 36 there is located a seat 38 upon which the second member 30 may reside. The seat 38 is preferably located around the periphery of the storage compartment 36 at a depth beneath the surface of the first member 18 that is equivalent to the height of the sidewalls 32 of the second member 30.

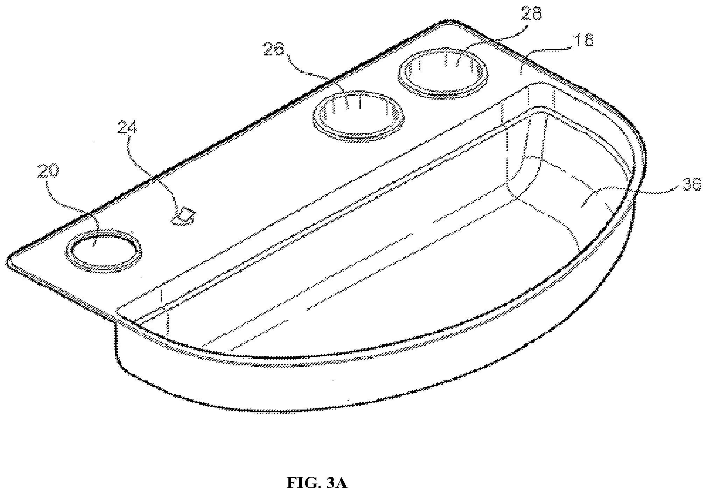

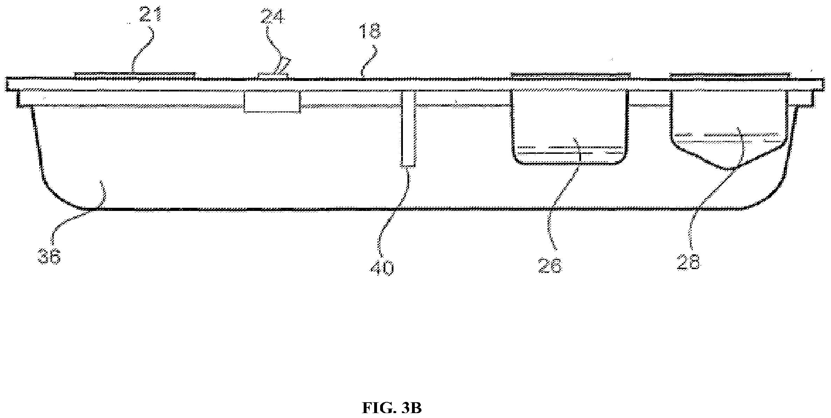

FIG. 3A provides a perspective view of the first member 18 showing the storage compartment 36, the heater activating switch 24, the aperture 20, the fluid containment well 26 and filter recess 28 formed within the first member. FIG. 3B provides a rear view of the first member 18 showing the filter recess 28, the fluid containment well 26, the heater activating switch 24, the aperture collar 21 and the rear wall of the storage compartment 36. As seen in this figure the filter recess has a conical base thereby providing a means for easy access to the cell suspension that is filtered into it. Located adjacent to the fluid containment well and on the opposite side of the filter containment well there is also provided a battery positioning member 40 which protrudes towards the base 16 of the apparatus (not shown) and provides a means for holding the batteries in place, which are required for activating the heating means.

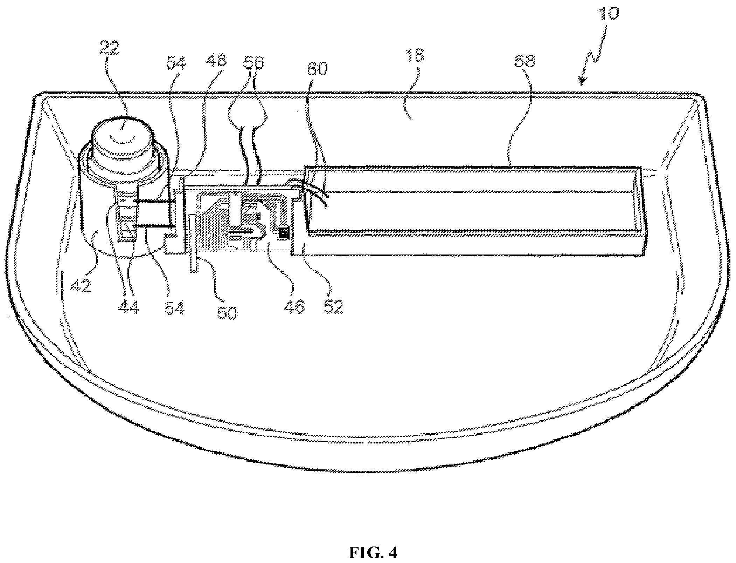

FIG. 4 provides a perspective view of the base 16 of the apparatus 10 showing the vial 22 located within a containment field 42. Between the containment field 42 and the vial 22 there is located a heating collar(s) 44, which surrounds the body of the vial. Adjacent the containment field there is a circuit board 46, which is held in position by circuit board containment means 48, 50 and 52. Said circuit board 46 is in electrical communications with the heating collar(s) 44 by wires 54. The circuit board is also in electrical communication via wires 58 with the heater activating switch (not shown). Adjacent the circuit board 46 there is provided a battery containment means 58, which holds 4 AA batteries in immovable position (not shown). The batteries are in electrical communication with the circuit board 46 by wires 60. When the first member 18 is fitted to the base 16 the batteries are held in place by the battery containment means 58, the battery positioning means 40 and the base of each of the fluid containment well 26 and the filter recess 28. Preferably the conical base of the filter recess 28 also protrudes between the batteries therein providing a further means for securing the batteries in immovable position.

### Example 3

It was tested whether HA hydrogels increase the viability and migration of human *stratum basale* epidermal Keratinocytes from adult donors. Various hydrogels were tested using assays including Transwell Cell Migration, CellTiter 96® Aqueous Cell Proliferation Assay and *in vitro* Scratch Assay described below.

### Protocol for Transwell Cell Migration

1. Maintain stock cultures of Primary Epidermal Keratinocytes (PEK), Normal, Human, Adult cells in Dermal Cell Basal Medium supplemented with Keratinocyte Growth Kit and Gentamicin-Amphotericin B Solution (Complete Media). Subculture the stock cultures of cells to 2.5 × 10⁴ cells/ml (estimate) and re-feed with media every 2 days or when a density of near confluence is reached.
2. To each well of a 24 well plate add 500 µl of Media. Inserts with an 8 um pore and 0.3 cm² area will be pre-coated with test material and placed into a well of a 24 well culture dish.
Row A, Columns 1-3: blank, no cells, medium only.
Row B, Columns 1-3: baseline, cells only, no coating.
Row C, Columns 1-3: Type 1 Collagen (positive control)
Row D, Columns 1-3, and Rows A-D, Columns 4-6: test substances in a serial dilution series.
(Illustrated below.)

| | Column 1 | Column 2 | Column 3 | Column 4 | Column 5 | Column 6 |
|---|---|---|---|---|---|---|
| Row A | Media Only | Media Only | Media Only | Media + HA (30 mg/ml) | Media + 1:1 HA/Collagen (30 mg/ml total polymer) | Media + Collagen (30 mg/ml) |
| Row B | Cells - No Coating | Cells - No Coating | Cells - No Coating | Media + HA (15 mg/ml) | Media +1:1 HA/Collagen (15 mg/ml total polymer) | Media + Collagen (15 mg/ml) |
| Row C | Type 1 Collagen - Positive Control | Type 1 Collagen - Positive Control | Type 1 Collagen - Positive Control | Media + HA (7.5 mg/ml) | Media + 1 : 1 HA/Collagen (7.5 mg/ml total polymer) | Media + Collagen (7.5 mg/ml) |
| Row D | | | | Media + HA (3.75 mg/ml) | Media + 1 :1 HA/Collagen (3.75 mg/ml total polymer) | Media + Collagen (3.75 mg/ml) |

3. Harvest cells and wash the PEK cells in Media by centrifugation at 150 × g for 3 to 5 minutes.
4. Determine cell number and viability (by trypan blue exclusion), and suspend the cells to a final concentration of 3 × 10⁵ cells/ml in Media.
5. Place inserts into plate.
6. Dispense 100µl of the cell suspension (30,000 cells) into all wells of the plate prepared in Step 2. The total volume in each well should be 165µl.
7. Incubate the plate for 6-12 hours at 37°C in a humidified, 5% CO2 atmosphere.
8. Gently wash filter with PBS, removing non-migratory cells on upper surface with a cotton swab.
9. Fix cells on lower surface with 4% paraformaldehyde for 2 hours.
10. Stain cells on lower surface with Harris haematoxylin for 30 mins.
11. For each membrane, count four randomly selected fields at 400X, determine average. Images may be taken of the membranes.

### Protocol for Cell Viability Testing using CellTiter 96® Aqueous Cell Proliferation Assay

1. Maintain stock cultures of Primary Epidermal Keratinocytes (PEK), Normal, Human, Adult cells in Dermal Cell Basal Medium supplemented with Keratinocyte Growth Kit and Gentamicin-Amphotericin B Solution (Complete Media). Subculture the stock cultures of cells to 2.5 × 10⁴ cells/ml (estimate) and re-feed with complete medium every 2 days or when a density of near confluence is reached.
2. Add 50µl/well of samples or standards to be measured, diluted in Complete Media.
   Row A, Columns 1-3: blank, no cells, medium only.
   Row B-C, Columns 1-3: baseline, cells only.
   Row D-H, Columns 1-3: sanguinarine, (toxin) dose response (50, 10, 5, 1, 0.1 µM)
   Row A-H Columns 4-6, 7-9, 8-12: test substances in a serial dilution series
   Equilibrate the plate at 37°C in a humidified, 5% CO2 atmosphere while harvesting the cells for assay.
3. Harvest cells and wash the PEK cells in Complete Media by centrifugation at 150 × g for 3 to 5 minutes.
4. Determine cell number and viability (by trypan blue exclusion), and suspend the cells to a final concentration of 6 × 10⁵ cells/ml in Complete Media.
5. Dispense 50 µl of the cell suspension (30,000 cells) into all wells of the plate prepared in Step 2. The total volume in each well should be 100 µl.
6. Incubate the plate for 48-72 hours at 37°C in a humidified, 5% CO2 atmosphere.
7. Add 20µl per well of MTS/PMS solution.
8. Incubate the plate for 1-4 hours at 37°C in a humidified, 5% CO2 atmosphere. To measure the amount of soluble formazan produced by cellular reduction of the MTS, proceed immediately to Step 9.
   Note: To measure the absorbance at a later time, add 25 µl of 10% SDS to each well to stop the reaction. Store SDS-treated plates protected from light in a humidified chamber at room temperature for up to 18 hours. Proceed to Step 9.
9. Record the absorbance at 490nm using an ELISA plate reader.
10. Plot the corrected absorbance at 490nm (Y axis) versus concentration of test products (X axis), and determine the ED50 value by determining the X-axis value corresponding to one-half the difference between the maximum (plateau) and minimum (no growth factor control) absorbance values. ED50 = the concentration of growth factor necessary to give one-half the maximal response.

Note: depending on response seen from test substrates, a cell/well standard curve may be performed to numerically estimate proliferation. This would be accomplished by plating a variety known number of viable cells per well and measuring fomazan production.

### Protocol for Cell Migration -Scratch Assay

1. Maintain stock cultures of Primary Epidermal Keratinocytes (PEK), Normal, Human, Adult cells in Dermal Cell Basal Medium supplemented with Keratinocyte Growth Kit and Gentamicin-Amphotericin B Solution (Complete Medium). Subculture the stock cultures of cells to 2.5 × 10⁴ cells/ml (estimate) and re-feed with complete medium every 2 days or when a density of near confluence is reached.
2. To each well of a 24 well plate add appropriate test substances.
   Row A, Columns 1-3: blank, no cells, medium only.
   Row B, Columns 1-3: baseline, cells only, no coating.
   Row C-D, Columns 1-3 and Rows A-D, Columns 4-6: test substances at determined previously, in a serial dilution series as necessary.
3. Harvest cells and wash the PEK cells in Media by centrifugation at 150 × g for 3 to 5 minutes.
4. Determine cell number and viability (by trypan blue exclusion), and suspend the cells to a final concentration of 5 × 10⁴ cells/ml in Media.
5. Dispense 600µl of the cell suspension (30,000 cells) into all wells of the plate prepared in Step 2.
6. Incubate the plate for 24 hours at 37°C in a humidified, 5% CO2 atmosphere.
7. With a pipette tip, scratch the monolayer of cells to create a cell-free zone in the center of the well.
8. Aspirate the spent medium and cells.
9. Wash with 300 µl of media to remove all cellular debris and loose cells.
10. Recoat the denuded surface with test substances (or media) for 1 hr at 37°C in a humidified, 5% CO2 atmosphere.
11. Aspirate spent media/test substances.
12. Wash cell layers once with media.
13. Replenish with 600 µl of medium.
14. Incubate the plate for 16 hours at 37°C in a humidified, 5% CO2 atmosphere. Images will be taken 16 hours after wounding.
Note: depending on results seen, cells may be pretreated with mitomycin C. This pre-treatment will assess the relative contribution of cell migration to *in vitro* wound closure in the absence of proliferation.

The cells utilized for all of the experiments were procured from the American Type Culture Collection (Catalog No. PCS-200-011, lot 59098517). These cells were primary epidermal keratinocytes from a normal adult human. Cells were maintained in Dermal Cell Basal Medium (ATCC No.: PCS 200-030) supplemented with keratinocyte growth kit (ATCC No. PCS 200-040) which contained bovine pituitary extract, recombinant human TGF-β, L-glutamine, hydrocortisone hemisuccinate, insulin, epinephrine, and apo-transferrin. A gentamicin-amphotericin B solution was also added to the medium (ATCC No.: 999-025). Cells were thawed, cultured and sub-cultured in accordance with ATCC recommendations.

Hyaluronic acid (HA) and NINJA™ hydrogels (hyaluronic acid with collagen) were prepared as follows.

Hyaluronic acid was prepared via a method described in U.S. Patent No. 6,660,853. In summary, raw hyaluronic acid sourced from rooster combs is dissolved in pure water at an estimated concentration of between 1 and 5 mg/ml. Using a Pall-Filtron 30 kDa MWCO PES membrane Type Centramate with an open channel configuration, the solution was then diafiltered against 5 volumes of pure pure water. The Membrane Type was an Omega Polyethersulfon Membrane with a filter area of 1.0 squar foot. A trans- Membrane Pressure (TMP) of no more than 8.0 psig was maintained, as well as a cross flow rate of at least 1,000 ml/min. The pump used to perform the diafiltration was a Cole- Parmer Masterflex® L/S.RTM. Precision Standard Tubing Pump capable of over 1700 ml/min, SKU# EW-77911-00. Sterilization was accomplished via Pall Bioinert Nylon 0.22 micron filters. Solution was then lyophilized and used in aseptic formulation using either 0.9% saline or cell medium as required. Solutions were initially made to 30 mg HA/ml ("HA30"), and then diluted as required (e.g., to 10 mg/mL, "HA10").

NINJA™ was formulated by using the hyaluronic acid purified above, and combining it with a native (non-cross linked) type I/III collagen in a method similar to U.S. Patent No. 7,371,399. Solution was then lyophilized and used in aseptic formulation using either 0.9% saline or cell medium as required. Solutions were initially made to 30 mg polymer/ml ("NINJA30"), and then diluted as required (e.g., to 10 mg/mL, "NINJA10").

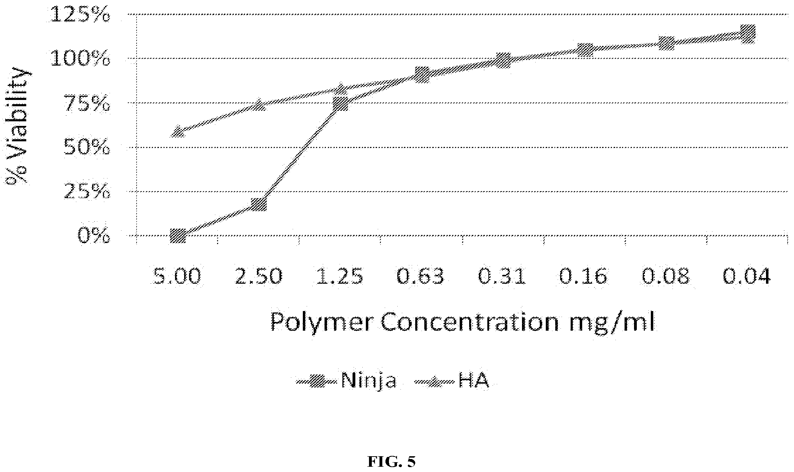

A commercially available kit [CellTiter, Promega] was purchased and used to test cell viability (MTS cell proliferation assay) according to the manufacturer's instructions. The assay is a colorimetric method for determining the activity of mitochondrial enzymes. Briefly, keratinocytes were seeded on 96-well plates at a density of 3x10^4 cells per well and incubated overnight with and without hydrogels. Twenty microlitres of CellTiter reagent containing tetrazolium compound and electron-coupling reagents were then added into each well to be catalysed by mitochondrial dehydrogenase enzymes in metabolically active cells. The plates were incubated for 1-4 h and the colorimetric absorbance was recorded at 490 nm by a microplate reader. In addition, readings were taken of blank wells and wells at full cell count. The wells of full cell count were read, blanks subtracted, and % viability was determined. Results are graphed in FIG. 5.

Reduction in Cell Viability in vitro at concentrations above 0.3 mg/ml of the HA hydrogels was observed. This is likely due to one of two situations. First, it is possible that the viscosity of the hydrogels reduces nutrient diffusion to the cells. This is supported by the added decrease in viability seen in the NINJA hydrogel at concentrations greater than 1.25 mg/ml. NINJA is more viscous at equivalent polymer concentrations than HA. Second, it is equally possible that the cells did not in fact die at higher polymer concentrations. It is just as likely that the dye, MTS, was not able to diffuse into the cells, and thus was not able to be processed into formazan via the mitochondrial reductase enzymes. This would also show up as a low absorbance in the 490-500 nm readings, and act as a false low viability reading. It should be noted though, that the molecular diffusion mechanism may not be the dominant mechanism *in vivo. In vivo* situations may involve enzymatic degradation, convective mass transfer (blood supply) and other as yet un-quantified mechanisms. Therefore, in *in vivo* settings, at least 2 times, 3 times, 4 times, 5 times, 6 times, 7 times, 8 times, 9 times, or higher concentrations of polymer may be tolerated than the 0.3 mg/mL limit in vitro.

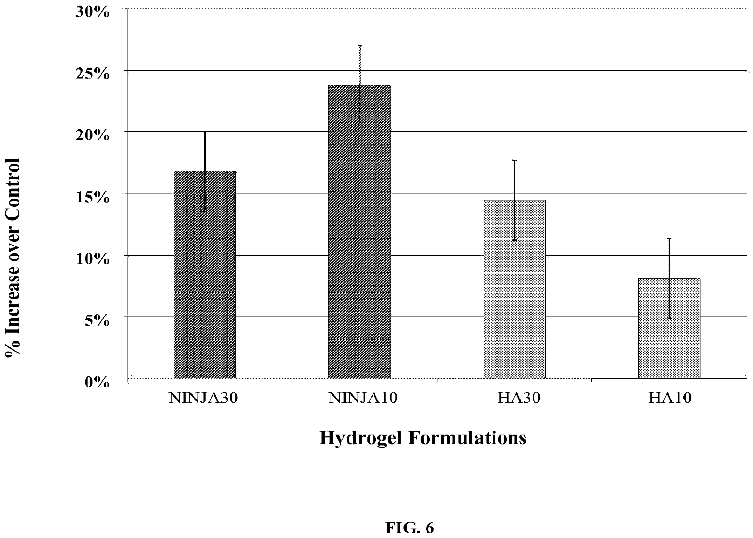

Trans-well Migration Assay was performed according to the above protocol. In summary, cell culture inserts equipped with an 8 um pore member of 0.3 cm² precoated with hydrogels were placed in 24-well culture dishes, forming the upper and lower compartments of the assay, respectively. The keratinocytes (3x10^4 per well) cultured in control medium were seeded onto the different hydrogel-coated top wells of varying viscosities and control medium and incubated for 3 days. All incubations were carried out under air/CO2 95%/5%, at37C. After the experiments, the filters were washed twice with PBS, and cells on the upper surface were removed using cotton swabs. Cells on the lower filter surface were fixed in 4% paraformaldehyde (2 h) and stained with Harris haematoxylin for 30 min. Cells migrating to the underside of membranes were counted in four randomly selected highpower fields, and the mean count was determined from duplicate experiments. Results are shown in FIG. 6. All hydrogel formulations outperform the control situation (media only), with NINJA10 leading.

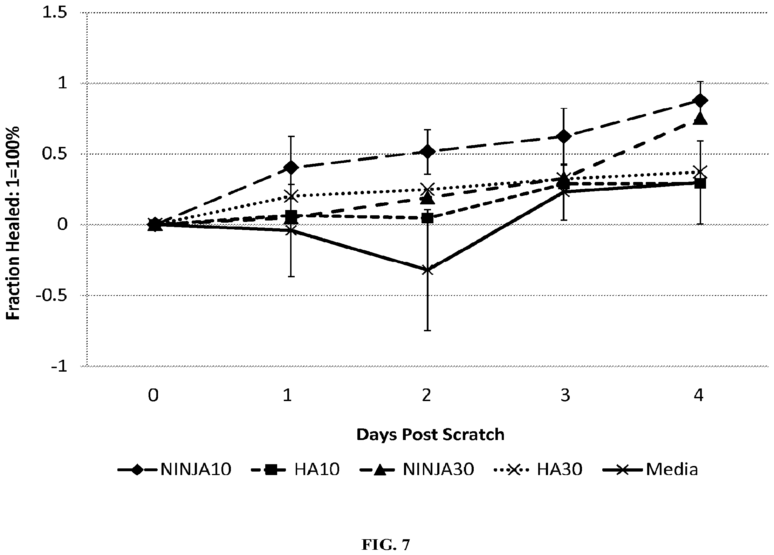

*In vitro* Scratch Assay was performed according to the above protocol. In summary, cultured keratinocytes (5x10^4 per mL) were plated in tissue culture dishes precoated with hydrogel of varying properties and nonhydrgel control and incubated overnight. The keratinocytes cultured in control medium were seeded on the different hydrogel -coated 24-well culture dishes for 3 days. Then the monolayer of cells was scratched in a standardized manner with a plastic apparatus to create a cell-free zone in each dish. The medium was then aspirated, followed by extensive washing to remove cellular debris. The denuded surfaces were recoated with hydrogel and keratinocyte culture medium for 1h at 37C. Then the culture medium containing hydrogel was aspirated and the cell layers were washed once. The culture dishes were replenished with fresh keratinocyte medium. Photographs were taken at 24 hour intervals after wounding for up to 4 days. *In vitro* re-epithelialization was documented by photography, and the amount of migration was manually quantified by computer-assisted image analysis. The data was expressed as a percentage of the area of the scratch filled by keratinocytes (FIG. 7). The results were obtained from the entire field of the "scratch", taken in duplicate. In addition, a second duplicate group of wells were tested with the added presence of mitomycin c (MMC+/-). Use of MMC slows cellular proliferation. Thus, differences in the healing of *in vitro* scratches with and without MMC can provide an indication on the hydrogel's role in migration versus mere proliferation and vice versa.

As shown in FIG. 7, of all the hydrogels tested, NINJA10 resulted in the highest healing. It was significantly better than the media control by day 2 and continued to be until the test was completed. The next best hydrogel was NINJA30. NINJA30 exhibited a delayed growth response. It was indistinguishable from the other hydrogels and media until the final day when it went from 33% to 75% in 24 hours. The higher viscosity of NINJA30 may have slowed the diffusion of necessary cellular nutrients until such time as the hydrogel had been diluted due to counter diffusion.

Differences in the healing of in vitro scratches with and without (+/-) MMC can provide an indication on the hydrogel's role in migration versus mere proliferation and vice versa. In the MMC- media/control group, the effect on healing was as expected. MMC+ cells showed delayed healing compared to MMC- cells, though the difference was not significant. This effect, however, was reversed in the HA30 group with MMC+ showing increased healing. HA10 MMC+ showed delayed healing for the first 3 days compared to HA10 MMC-, but then reversed on the fourth day. NINJA30 and NINJA10 MMC+ also showed slight delays in healing.

Thus, the hydrogels act to up regulate both cell proliferation and migration. Given the general trend of all hydrogels contributing to healing, and the significance of the NINJA10 group (and part of the NINJA30 group), NINJA can be an appropriate technology to augment ReCell® and wound healing.

As discussed above, the dominant mechanism in any in vitro system is molecular diffusion. Media nutrients diffuse into hydrogels, and the polymers diffuse out of the hydrogels over time. This is referred to as counter diffusion. In vivo conditions add to the complexity of any model by introducing additional mechanisms including forced convection (blood flow) and enzymatic degradation. Therefore, it is possible that even though the above testing suggests that 0.3 mg of polymer/ml is the viability limit, this is only true of in vitro systems. The viability limits in vivo is likely higher. Indeed, the NINJA™ technology has been injected into patients extensively over the last several years, especially in the temporomandibular joint which contains a variety of chondrocytes, fibroblasts and other cell populations. Injections of 30 mg polymer/ ml showed significant increases in healing. Therefore, higher polymer gel concentrations in the range that appears to compromise cell viability in vitro may be tolerated in vivo.

The initial positive results suggest a solution having a higher viscosity as vehicle for the application of autologous cells. The solutions can include formulation NINJA 10 or a variant thereof.

Modifications and variations of the described methods and compositions of the invention will be apparent to those skilled in the art. The invention has been described in connection with specific preferred embodiments.

## Claims

1. A cell suspension composition for use in an epithelium-related procedure, comprising:
a population of cells including viable and functioning cells derived from an epithelial tissue sample; and
an exogenous agent comprising hyaluronic acid;
wherein the population of cells, prior to being used in an epithelium-related procedure, are not subject to in vitro culturing and comprise both stem cells and non-stem cells;
wherein the population of cells, when used in said epithelium-related procedure and applied to a recipient site, promote treatment, healing, reconstructing, resurfacing, repigmentation and/or regeneration of epithelial tissues.

2. The cell suspension composition of claim 1, wherein the epithelial tissue sample is obtained from one or more of skin epithelium, respiratory epithelium, vascular epithelium, corneal epithelium, and glandular epithelium.

3. The cell suspension composition of claim 1 or 2, wherein when the epithelial tissue sample is skin tissue sample,
(1) the viable cells are derived from a dermal layer, epidermal layer, or both a dermal and an epidermal layer of the skin tissue sample;
(2) preferably the population of cells comprises keratinocytes, melanocytes, fibroblasts, Langerhans cells, or any combination of the foregoing;
(3) preferably the population of cells comprises skin stem cells, wherein preferably the skin stem cells are combined with non-stem cells; and/or
(4) preferably the epithelium-related procedure comprises skin wound treatment, skin tissue graft, skin aesthetic procedure, or dermal treatment, wherein preferably the skin wound is acute wound, artificially created wound, or chronic wound.

4. The cell suspension composition of any one of claims 1-3, wherein the population of cells are seeded to a matrix or scaffold in vitro or in situ at a recipient site, for use in said epithelium-related procedure.

5. The cell suspension composition of any one of claims 1-4, wherein the exogenous agent is hyaluronic acid or hyaluronic acid with collagen.

6. A method for preparing cells for use in an epithelium-related procedure, comprising:
providing a population of cells including viable and functioning cells derived from an epithelial tissue sample; and
exposing the population of cells to an exogenous agent comprising hyaluronic acid;
wherein the population of cells, prior to being used in an epithelium-related procedure, are not subject to in vitro culturing and comprise both stem cells and non-stem cells.

7. The method of claim 6, wherein the epithelial tissue sample is obtained from one or more of skin epithelium, respiratory epithelium, vascular epithelium, corneal epithelium, and glandular epithelium.

8. The method of claim 6 or 7, wherein when the epithelial tissue sample is skin tissue sample, (1) the viable cells are derived from a dermal layer, epidermal layer, or both a dermal and an epidermal layer of the skin tissue sample;
(2) preferably the population of cells comprises keratinocytes, melanocytes, fibroblasts, Langerhans cells, or any combination of the foregoing;
(3) preferably the population of cells comprises skin stem cells, wherein preferably the skin stem cells are combined with non-stem cells; and/or
(4) preferably the epithelium-related procedure comprises skin wound treatment, skin tissue graft, skin aesthetic procedure, or dermal treatment, wherein preferably the skin wound is acute wound, artificially created wound, or chronic wound.

9. The method of any one of claims 6-8, further comprising seeding the population of cells to a matrix or scaffold in vitro or in situ at a recipient site.

10. The method of any one of claims 6-9, wherein the exogenous agent is hyaluronic acid or hyaluronic acid with collagen.

## Patentansprüche

1. Eine Zellsuspensionszusammensetzung zur Verwendung in einem epithelbezogenen Verfahren, das Folgendes beinhaltet:
eine Population von Zellen, die lebensfähige und
funktionierende Zellen umfasst, die aus einer Epithelgewebeprobe gewonnen wurden; und
ein exogenes Mittel, das Hyaluronsäure beinhaltet;
wobei die Population von Zellen vor ihrer Verwendung in einem epithelbezogenen Verfahren keiner In-vitro-Kultivierung unterzogen wird und sowohl Stammzellen als auch Nicht-Stammzellen beinhaltet;
wobei die Population von Zellen, wenn sie in dem epithelbezogenen Verfahren verwendet wird und auf einen Empfängerort appliziert wird, Behandlung, Heilung, Rekonstruktion, Resurfacing, Repigmentierung und/oder Regeneration von Epithelgewebe fördert.

2. Zellsuspensionszusammensetzung gemäß Anspruch 1, wobei die Epithelgewebeprobe von einem oder mehreren der Folgenden erhalten wird: Hautepithel, Atemepithel, Gefäßepithel, Korneaepithel und Drüsenepithel.

3. Zellsuspensionszusammensetzung gemäß Anspruch 1 oder 2, wobei, wenn die Epithelgewebeprobe eine Hautgewebeprobe ist,
(1) die lebensfähigen Zellen von einer dermalen Schicht, epidermalen Schicht oder sowohl von einer dermalen als auch einer epidermalen Schicht der Hautgewebeprobe gewonnen werden;
(2) die Population von Zellen bevorzugt Keratinozyten, Melanozyten, Fibroblasten, Langerhans-Zellen oder eine beliebige Kombination der zuvor genannten beinhaltet;
(3) die Population von Zellen bevorzugt Hautstammzellen beinhaltet, wobei die Hautstammzellen bevorzugt mit Nicht-Stammzellen kombiniert sind; und/oder
(4) das epithelbezogene Verfahren bevorzugt eine Hautwundbehandlung, ein Hautgewebetransplantat, ein ästhetisches Hautverfahren oder eine dermale Behandlung beinhaltet, wobei die Hautwunde bevorzugt eine akute Wunde, künstlich erzeugte Wunde oder chronische Wunde ist.

4. Zellsuspensionszusammensetzung gemäß einem der Ansprüche 1-3, wobei die Population von Zellen in vitro oder in situ an einem Empfängerort auf eine Matrix oder ein Gerüst zur Verwendung in dem epithelbezogenen Verfahren gesät werden.

5. Zellsuspensionszusammensetzung gemäß einem der Ansprüche 1-4, wobei das exogene Mittel Hyaluronsäure oder Hyaluronsäure mit Kollagen ist.

6. Ein Verfahren zum Vorbereiten von Zellen zur Verwendung in einem epithelbezogenen Verfahren, das Folgendes beinhaltet:
Bereitstellen einer Population von Zellen, die lebensfähige und funktionierende Zellen umfasst, die aus einer Epithelgewebeprobe gewonnen sind; und
Exponieren der Population von Zellen gegenüber einem exogenen Mittel, das Hyaluronsäure beinhaltet;
wobei die Population von Zellen vor ihrer Verwendung in einem epithelbezogenen Verfahren keiner In-vitro-Kultivierung unterzogen wird und sowohl Stammzellen als auch Nicht-Stammzellen beinhaltet.

7. Verfahren gemäß Anspruch 6, wobei die Epithelgewebeprobe von einem oder mehreren der Folgenden erhalten wird: Hautepithel, Atemepithel, Gefäßepithel, Korneaepithel und Drüsenepithel.

8. Verfahren gemäß Anspruch 6 oder 7, wobei, wenn die Epithelgewebeprobe eine Hautgewebeprobe ist, (1) die lebensfähigen Zellen von einer dermalen Schicht, epidermalen Schicht oder sowohl von einer dermalen als auch einer epidermalen Schicht der Hautgewebeprobe gewonnen werden;
(2) die Population von Zellen bevorzugt Keratinozyten, Melanozyten, Fibroblasten, Langerhans-Zellen oder eine beliebige Kombination der zuvor genannten beinhaltet;
(3) die Population von Zellen bevorzugt Hautstammzellen beinhaltet, wobei die Hautstammzellen bevorzugt mit Nicht-Stammzellen kombiniert sind; und/oder
(4) das epithelbezogene Verfahren bevorzugt eine Hautwundbehandlung, ein Hautgewebetransplantat, ein ästhetisches Hautverfahren oder eine dermale Behandlung beinhaltet, wobei die Hautwunde bevorzugt eine akute Wunde, künstlich erzeugte Wunde oder chronische Wunde ist.

9. Verfahren gemäß einem der Ansprüche 6-8, das ferner das Säen der Population von Zellen in vitro oder in situ an einem Empfängerort auf eine Matrix oder ein Gerüst beinhaltet.

10. Verfahren gemäß einem der Ansprüche 6-9, wobei das exogene Mittel Hyaluronsäure oder Hyaluronsäure mit Kollagen ist.

## Revendications

1. Composition de suspension de cellules destinée à être utilisée dans une intervention relative à l'épithélium, comprenant :
une population de cellules renfermant des cellules viables et fonctionnelles issues d'un échantillon de tissu épithélial ; et
un agent exogène comprenant de l'acide hyaluronique ;
dans laquelle la population de cellules, avant d'être utilisée dans une intervention relative à l'épithélium, n'est pas soumise à une culture *in vitro* et comprend à la fois des cellules souches et des cellules qui ne sont pas des cellules souches ;
dans laquelle la population de cellules, lorsqu'elle est utilisée dans ladite intervention relative à l'épithélium et appliquée sur un site receveur, favorise le traitement, la cicatrisation, la reconstruction, le resurfaçage, la repigmentation et/ou la régénération de tissus épithéliaux.

2. Composition de suspension de cellules selon la revendication 1, dans laquelle l'échantillon de tissu épithélial est obtenu à partir d'épithélium cutané, d'épithélium respiratoire, d'épithélium vasculaire, d'épithélium cornéen et/ou d'épithélium glandulaire.

3. Composition de suspension de cellules selon la revendication 1 ou 2, dans laquelle lorsque l'échantillon de tissu épithélial est un échantillon de tissu cutané,
(1) les cellules viables sont issues d'une couche dermique, d'une couche épidermique ou à la fois d'une couche dermique et d'une couche épidermique de l'échantillon de tissu cutané ;
(2) de préférence la population de cellules comprend des kératinocytes, des mélanocytes, des fibroblastes, des cellules de Langerhans ou une quelconque combinaison de celles-ci ;
(3) de préférence la population de cellules comprend des cellules souches cutanées, dans laquelle de préférence les cellules souches cutanées sont combinées avec des cellules qui ne sont pas des cellules souches ; et/ou
(4) de préférence l'intervention relative à l'épithélium comprend un traitement de plaie cutanée, une greffe de tissu cutané, une intervention esthétique cutanée ou un traitement dermique, dans laquelle de préférence la plaie cutanée est une plaie aiguë, une plaie créée artificiellement ou une plaie chronique.

4. Composition de suspension de cellules selon l'une quelconque des revendications 1-3, dans laquelle la population de cellules est ensemencée sur une matrice ou un échafaudage *in vitro* ou *in situ* au niveau d'un site receveur, destiné à être utilisé dans ladite intervention relative à l'épithélium.

5. Composition de suspension de cellules selon l'une quelconque des revendications 1-4, dans laquelle l'agent exogène est l'acide hyaluronique ou l'acide hyaluronique avec du collagène.

6. Procédé pour la préparation de cellules destinées à être utilisées dans une intervention relative à l'épithélium, comprenant :
la fourniture d'une population de cellules renfermant des cellules viables et fonctionnelles issues d'un échantillon de tissu épithélial ; et
l'exposition de la population de cellules à un agent exogène comprenant de l'acide hyaluronique ;
dans lequel la population de cellules, avant d'être utilisée dans une intervention relative à l'épithélium, n'est pas soumise à une culture *in vitro* et comprend à la fois des cellules souches et des cellules qui ne sont pas des cellules souches.

7. Procédé selon la revendication 6, dans lequel l'échantillon de tissu épithélial est obtenu à partir d'épithélium cutané, d'épithélium respiratoire, d'épithélium vasculaire, d'épithélium cornéen et/ou d'épithélium glandulaire.

8. Procédé selon la revendication 6 ou 7, dans lequel lorsque l'échantillon de tissu épithélial est un échantillon de tissu cutané,
(1) les cellules viables sont issues d'une couche dermique, d'une couche épidermique ou à la fois d'une couche dermique et d'une couche épidermique de l'échantillon de tissu cutané ;
(2) de préférence la population de cellules comprend des kératinocytes, des mélanocytes, des fibroblastes, des cellules de Langerhans ou une quelconque combinaison de celles-ci ;
(3) de préférence la population de cellules comprend des cellules souches cutanées, dans lequel de préférence les cellules souches cutanées sont combinées avec des cellules qui ne sont pas des cellules souches ; et/ou
(4) de préférence l'intervention relative à l'épithélium comprend un traitement de plaie cutanée, une greffe de tissu cutané, une intervention esthétique cutanée ou un traitement dermique, dans lequel de préférence la plaie cutanée est une plaie aiguë, une plaie créée artificiellement ou une plaie chronique.

9. Procédé selon l'une quelconque des revendications 6-8, comprenant en outre l'ensemencement de la population de cellules sur une matrice ou un échafaudage *in vitro* ou *in situ* au niveau d'un site receveur.

10. Procédé selon l'une quelconque des revendications 6-9, dans lequel l'agent exogène est l'acide hyaluronique ou l'acide hyaluronique avec du collagène.
